# EUROPEAN PATENT APPLICATION

(11) **EP 2 905 337 A1**
(43) Date of publication of application: **12.08.2015**
(21) Application number: 13843342.0
(22) Date of filing: 07.10.2013
(51) Int. Cl.: C12N 15/113, A61K 31/7105

(54) **AMPHIREGULIN-SPECIFIC DOUBLE-HELICAL OLIGO-RNA, DOUBLE-HELICAL OLIGO-RNA STRUCTURE COMPRISING DOUBLE-HELICAL OLIGO-RNA, AND COMPOSITION FOR PREVENTING OR TREATING RESPIRATORY DISEASES CONTAINING SAME**

(30) Priority: 05.10.2012 KR 20120110559
(71) Applicant: Bioneer Corporation, Daejon 306-220 (KR)
(72) Inventor: PARK, Han-Oh, Daejeon 305-761 (KR); CHAE, Jeiwook, Daejeon 305-762 (KR); YOON, Pyoung Oh, Daejeon 302-743 (KR)
(74) Representative: Held, Stephan
(86) International application number: PCT/KR2013/008949
(87) International publication number: WO 2014/054927

(57) **Abstract**

The present invention relates to an amphiregulin-specific double-stranded oligo siRNA, which can inhibit the expression of amphiregulin with high efficiency and can be effectively used for the prevention or treatment of respiratory diseases. Moreover, the present invention relates to an amphiregulin-specific double-stranded oligo siRNA structure that comprises hydrophilic and hydrophobic compounds bound to the amphiregulin-specific double-stranded oligo siRNA by a simple covalent bond or a linker-mediated covalent bond so as to increase the in vivo stability and intracellular delivery efficiency of the double-stranded oligo siRNA, and to nanoparticles composed of the oligo siRNA structures. The amphiregulin-specific double-stranded oligo siRNA structure according to the present invention can increase the stability of the amphiregulin-specific double-stranded oligo siRNA without impairing the function of the amphiregulin-specific double-stranded oligo siRNA, and at the same time, can efficiently increase the membrane permeability of the double-stranded oligo siRNA. Thus, when the amphiregulin-specific double-stranded oligo siRNA structure is used, the amphiregulin-specific double-stranded oligo siRNA can be efficiently delivered into cells even when it is administered at a relatively low concentration, so that it can be effectively used for the prevention or treatment of respiratory diseases.

## Description

### TECHNICAL FIELD

The present invention relates to a respiratory disease-related gene-specific double-stranded oligo RNA and a high-efficiency double-stranded oligo RNA structure comprising the same, in which the double-stranded oligo RNA structure comprises a hydrophilic compound and hydrophobic compound conjugated to both ends of the double-stranded RNA (siRNA) by a single covalent bond or a linker-mediated covalent bond so that it will be efficiently delivered into cells, and can be converted into nanoparticles by hydrophobic interactions between the double-stranded oligo RNA structures in an aqueous solution. The double-stranded oligo RNA included in the double-stranded oligo RNA structure is preferably specific for amphiregulin, a respiratory disease-related gene, and is particularly preferably an amphiregulin-specific siRNA.

Moreover, the present invention relates to a method for preparing the double-stranded oligo RNA structure, and a pharmaceutical composition for preventing or treating respiratory diseases, particularly chronic obstructive pulmonary disease (COPD) and/or idiopathic pulmonary fibrosis (IPF), which comprises the double-stranded oligo RNA structure.

### BACKGROUND ART

A technology for inhibiting gene expression is an important tool in the development of therapeutic agents for treating diseases and in target verification. As conventional technologies for inhibiting the expression of a target gene, technologies for introducing a transgene for a target gene have been disclosed, including a method of introducing a transgene in an antisense direction with respect to the promoter (Sheehy et al., Proc. Natl. Acad. Sci., USA, 85:8805-8808, 1988; Smith et al., Nature, 334:724-726, 1988), and a method of introducing a transgene in a sense direction with the respect to the promoter (Napoli et al., Plant Cell, 2:279-289, 1990; van der Krol et al., Plant Cell, 2:291-299, 1990; US Patent No. 5,034,323; US Patent No. 5,231,020; and US Patent No. 5,283,184).

Meanwhile, it was recently reported that post-translational gene silencing or RNA interference (RNAi) is caused by the accumulation of a double-stranded RNA fragment having 20-25 base pairs, and the double-stranded RNA is synthesized from an RNA template (Hamilton & Baulcumbe, Science, 286:950-952, 1999). The double-stranded RNA fragment was named "small interfering RNA (hereinafter referred to as siRNA)". Since then, it has been demonstrated that siRNA is an important factor that inhibits the expression of genes in a variety of living organisms, including mammals (Fire et al., Nature, 391:806-811, 1998; Timmons & Fire, Nature, 395:854, 1998; Kennerdell & Carthew, Cell, 95:1017-1026, 1998; Elbashir et al., Nature, 411:494-497, 2001; WO 99/32619). In addition, it is known that a double-stranded siRNA is converted to a single-stranded RNA by a RISC (RNA-induced silencing complex) protein complex in cells, and then binds to and inactivate a target mRNA (Novina & Sharp, Nature, 430:161-164, 2004). The technology for inhibiting the expression of a gene using an siRNA as described above is used to inhibit the expression of a target gene in target cells and to observe changes caused thereby and is also advantageously used in studies focused on identifying the functions of a target gene in target cells. In particular, it is expected that inhibiting the function of a target gene in infectious virus or cancer cells can be effectively used in the development of a method for treating the relevant disease. The results of *in vitro* studies and *in* vivo studies conducted using experimental animals indicated that the silencing of a target gene by siRNA is possible (McCaffrey et al., Nature Biotechnology, 21:639-644, 2003; Giladi et al., Molecular Therapy, 8:769-776, 2003; Konishi et al., epatology, 38:842-850, 2003; Sen et al., Virus Research, 96:27-35, 2003; Yokota et al., EMBO Reports, 4:602-608, 2003; Song et al., Nature Medicine, 9:347-351, 2003; McCaffrey et al., Nature, 418:38-39, 2002). For example, International Patent Publication No. WO 03/070969 discloses a method of treating cancer cells by inhibiting the expression of Bcl2 protein in the cancer cells using siRNA, and International Patent Publication No. WO 04/009769 discloses a method of treating cancer cells by inhibiting the expression of an angiogenic VEGF protein using siRNA.

Also, because siRNA complementarily binds to the target mRNA to regulate the expression of the target gene in a sequence-specific manner, it can be advantageously used in a remarkably wide range of applications compared to conventional antibody-based drugs or chemicals (small molecule drugs) (Progress Towards in Vivo Use of siRNAs. MOLECULAR THERAPY. 2006 13(4):664-670).

siRNA has excellent effects and can be used in a wide range of applications, but in order for siRNA to be developed into therapeutic agents, it is required to improve the *in vivo* stability and intracellular delivery efficiency of siRNA so as to effectively deliver siRNA into its target cells (Harnessing in vivo siRNA delivery for drug discovery and therapeutic development. Drug Discov Today. 2006 Jan; 11(1-2):67-73).

In order to improve the *in vivo* stability of siRNA and solve problems associated with the innate immune stimulation of siRNA, studies on a technology of either modifying some nucleotides of siRNA or the backbone of siRNA to improve the *in vivo* stability so as to have resistance against nuclease or using carriers such as a viral vector, liposome or nanoparticles have been actively conducted.

Delivery systems comprising a viral vector such as adenovirus or retrovirus have high transfection efficiency, but carry the risks of immunogenicity and oncogenicity. On the other hand, non-viral carriers including nanoparticles are evaluated to have low intracellular delivery efficiency compared to viral carriers, but have advantages, including high safety *in vivo,* target-specific delivery, efficient uptake and internalization of RNAi oligonucleotides into cells or tissues, and low cytotoxicity and immune stimulation. Thus, these non-viral carriers are considered as a promising delivery method compared to the vial delivery system (Nonviral delivery of synthetic siRNAs in vivo. J Clin Invest. 2007 December 3; 117(12): 3623-3632).

In a method of using nanocarriers among the non-viral delivery systems, nanoparticles are formed using various polymers such as liposome, a cationic polymer complex and the like, and siRNA is supported on these nanoparticles (i.e., nanocarriers), and is delivered into the cell. In the method of using nanocarriers, a polymeric nanoparticle, polymer micelle, lipoplex and the like are mainly used. Among them, the lipoplex is composed of cationic lipid and interacts with anionic lipid of endosome in the cell to destabilize the endosome, thereby delivering the siRNA into the cell (Proc. Natl. Acad. Sci. 15; 93(21):11493-8, 1996).

In addition, it is known that the efficiency of siRNA *in vivo* can be increased by conjugating a chemical compound or the like to the end region of the passenger (sense) strand of the siRNA so as to have improved pharmacokinetic characteristics (Nature 11; 432(7014):173-8, 2004). In this case, the stability of the siRNA changes depending on the property of the chemical compound conjugated to the end of the sense (passenger) or antisense (guide) strand of the siRNA. For example, siRNA conjugated with a polymer compound such as polyethylene glycol (PEG) interacts with the anionic phosphoric acid group of siRNA in the presence of a cationic compound to form a complex, thereby providing a carrier having improved siRNA stability (J Control Release 129(2):107-16, 2008). Particularly, micelles made of a polymer complex have a very small size and a very uniform size distribution compared to other drug delivery systems such as microspheres or nanoparticles, and are spontaneously formed. Thus, these micelles have advantages in that the quality of the formulation is easily managed and the reproducibility thereof is easily secured.

Further, in order to improve the intracellular delivery efficiency of siRNA, a technology for securing the stability of the siRNA and increasing the cell membrane permeability of the siRNA using a siRNA conjugate obtained by conjugating a hydrophilic compound (for example, polyethylene glycol (PEG)), which is a biocompatible polymer, to the siRNA via a simple covalent bond or a linker-mediated covalent bond, has been developed (Korean Patent Registration No. 883471). However, even when the siRNA is chemically modified and conjugated to polyethylene glycol (PEG) (PEGylation), it still has low stability *in vivo* and a disadvantage in that it is not easily delivered into a target organ. In order to solve these disadvantages, double-stranded oligo RNA structures comprising hydrophilic and hydrophobic compounds bound to an oligonucleotide, particularly double-stranded oligo RNA such as siRNA, have been developed. These structures form self-assembled nanoparticles, named SAMiRNA™ (Self Assembled Micelle Inhibitory RNA), by hydrophobic interaction of the hydrophobic compound (see Korean Patent Registration No. 1224828). The SAMiRNA™ technology has advantages over conventional delivery technologies in that homogenous nanoparticles having a very small size can be obtained.

Meanwhile, it is known that amphiregulin binds to epidermal growth factor receptor to activate the epidermal growth factor receptor (EGFR) pathway and is involved in cell proliferation. It was reported that the expression of amphiregulin can be inhibited by an amphiregulin-specific siRNA, and this inhibition of expression of amphiregulin exhibits a therapeutic effect against a specific type of breast cancer (Cancer Res. 2008; 68:225-2265). Further, it was reported that cell infiltration in inflammatory breast cancer can be inhibited by the use of an amphiregulin-specific shRNA (J Cell Physiol. 2011 226(10):2691-2701), and when the expression of amphiregulin is inhibited using an amphiregulin-specific shRNA, pulmonary artery remodeling in rats exposed to cigarette smoke is inhibited (Arch Biochem Biophys, 1;508(1):93-100). It is known that amphiregulin is involved in airway smooth muscle (ASM) hyperplasia and angiogenesis, and particularly, amphiregulin stimulates airway remodeling in asthma patients (J Korean Med Sci 2008; 23: 857-863), and amphiregulin and epidermal growth factor (EGF) that is excessively secreted in tissue remodeling caused by acute asthma are involved therein (J Alergy Clin Immunol 2009;124:913-920).

Chronic obstructive pulmonary disease (hereinafter referred to as 'COPD'), a typical pulmonary disease together with asthma, differs from asthma in that it involves irreversible airway obstruction. It is a respiratory disease that shows an airflow limitation, which is progressive and not fully reversible and is associated with an abnormal inflammatory response of the lungs, which is caused by repeated infections, noxious gas or gas inhalation or cigarette smoking (Am J Respir Crit Care Med, 163:1256-1276, 2001). The severity of COPD has been emphasized worldwide. COPD was the sixth leading cause of death in 1990, but is expected to be the third leading cause of death in 2020. It is the only disease among the top 10 causes of death with rising incidence. Also, it is expected to be the fourth leading cause of injury-related hospitalization, and thus the social and economic burdens caused by COPD will increase rapidly (Lancet, 349:1498-1504, 1997). Chronic obstructive pulmonary disease (COPD) is caused by pathological changes in bronchioles and parenchyma due to inflammations of the airway and parenchyma, and is characterized by bronchiolitis obliterans and emphysema (parenchymal destruction). Examples of chronic obstructive pulmonary disease include chronic obstructive bronchitis, chronic bronchiolitis and emphysema. In the case of chronic obstructive pulmonary disease, the number of neutrophils increases, and cytokines such as GM-CSF, TNF-α, IL-8 or MIP-2 are secreted. In addition, inflammation occurs in the airway, the muscular wall becomes thicker, and mucus secretion increases to cause airway obstruction. When the airway is obstructed, alveoli will be enlarged and damaged so that its ability to exchange oxygen with carbon dioxide will be impaired and the development of respiratory failure will increase. 8% of adults aged 45 or older in Korea is chronic obstructive pulmonary disease patients, but interest has been focused on lung cancer. In conventional methods for treating chronic obstructive pulmonary disease, either therapeutic agents having anti-inflammatory activity or therapeutic agents having bronchodilating effects are mainly used, and the fundamental prevention and treatment of obstructive pulmonary disease by gene therapeutic agents are insignificant. Representative examples of the therapeutic agent having anti-inflammatory activity include glucocorticoid, leukotriene modifiers, theophylline and the like. Among them, glucocorticoid has potent effects, but is administered by inhalation due to its side effects, and it does not selectively inhibit inflammatory reactions, but inhibits all immune responses, and thus inhibits even necessary immune responses in some cases. Leukotriene modifiers have less side effects, but have limited effects so that they cannot control asthma when being used alone. For this reason, leukotriene modifiers are mostly as used as auxiliaries. Theophylline has advantages in that the effect thereof is not excellent and it may cause side effects. Thus, there is an urgent demand for a new therapeutic agent for preventing and treating chronic obstructive pulmonary disease, which has excellent effects and causes less side effects.

Meanwhile, idiopathic pulmonary fibrosis (hereinafter referred to as 'IPF') is a disease in which chronic inflammatory cells penetrate the alveolus wall while various changes making the lung rigid occur to cause a severe structural change in the lung tissue so that the function of the lung will be progressively impaired, leading to death. An effective method for treating IPF has not yet been known, and patients with IPF have a poor prognosis with a median survival time of about 305 years after diagnosis. It was reported that the incidence of IPF is about 3-5 persons per 100,000 persons in foreign countries. Also, it is known that IPF occurs in persons aged over 50, and the incidence thereof is higher in men than in women.

The cause of development of IPF has not yet been clearly identified, and it was reported that the incidence of IPF is high in smokers, and anti-depressants, chronic pulmonary inhalation by gastroesophageal reflux, the inhalation of metal dust, wood dust or solvents, etc., are risk factors associated with the development of IPF. However, factors having a certain causal relation cannot be found in most IPF patients.

It is known that, when IPF is not treated, it continuously grows worse, and about 50% or more of IPF patients die within 3-5 years after diagnosis. Also, after the disease completely progresses to fibrosis, it does not get better even by any treatment. Thus, when IPF is treated at an early stage, the possibility of treating IPF will be high. As therapeutic agents for treating IPF, combinations of steroid and azathioprine or cyclophosphamide are currently known, but do not appear to exhibit special effects. In addition, various fibrosis inhibitors have been have been attempted in animal tests and a small number of patients, but the distinct effects thereof have not been proven. Particularly, therapeutic methods other than lung transplantation for end-stage IPF patients have not been reported. Therefore, there is an urgent need for the development of a therapeutic agent for more efficient treatment of IPF.

### [Prior art literature]

### [Patent documents]

Patent document 1: U.S. Pat. No. 5,034,323
Patent document 2: U.S. Pat. No. 5,231,020
Patent document 3: U.S. Pat. No. 5,283,184
Patent document 4: Korean Patent Registration No. 883471

### [Non-Patent documents]

Non-Patent Document 1: Proc. Natl. Acad. Sci., USA, 85:8805-8808, 1988
Non-Patent Document 2: Smith et al., Nature, 334:724-726, 1988
Non-Patent Document 3: Plant Cell, 2:279-289, 1990
Non-Patent Document 4: Plant Cell, 2:291-299, 1990

### DISCLOSURE OF INVENTION

The present invention has been made in order to solve the above-described problems occurring in the prior art, and it is an object of the present invention to provide a novel double-stranded oligo RNA, particularly siRNA, which can inhibit the expression of amphiregulin with very high efficiency in a manner specific for amphiregulin, a double-stranded oligo RNA structure comprising the novel oligo RNA, and a method for preparing the double-stranded oligo RNA structure.

Another object of the present invention is to provide a pharmaceutical composition for preventing or treating respiratory diseases, particularly COPD and/or IPF, which comprises, as an active ingredient, the amphiregulin-specific double-stranded oligo RNA or the double-stranded oligo RNA structure comprising the double-stranded oligo RNA

Still another object of the present invention is to provide a pharmaceutical composition of treating respiratory diseases, particularly COPD and/or IPF, by using the amphiregulin-specific double-stranded oligo RNA or the double-stranded oligo RNA structure comprising the double-stranded oligo RNA.

Other features and embodiments of the present invention will be more apparent from the following detailed descriptions and the appended claims.

### BRIEF DESCRIPTION OF THE DRAWINGS

FIG. 1 is a graph showing the relative expression level (%) of amphiregulin mRNA in transformed cells treated with 20 nM of each of 30 different siRNAs comprising a sense strand having each of sequences of SEQ ID NOS: 1 to 30.
   NC: the expression level of amphiregulin mRNA in a total RNA obtained from cells (control) not treated with an amphiregulin-specific siRNA. The relative expression level of amphiregulin caused by each siRNA was measured relative to NC taken as 100%.
FIG. 2 is a graph showing the relative expression level (%) of amphiregulin mRNA in fibroblast cell lines treated with varying concentrations (0.2, 1 and 5 nM) of siRNAs comprising a sense strand having each of sequences of SEQ ID NOS: 8, 9 and 28.
FIG. 3 is a graph showing the relative expression level (%) of amphiregulin mRNA in transformed cells treated with 20 nM of each of siRNAs comprising a sense strand having each of sequences of SEQ ID NOS: 31 to 50.

### BEST MODE FOR CARRYING OUT THE INVENTION

Unless defined otherwise, all technical and scientific terms used herein have the same meaning as commonly understood by one of ordinary skill in the art to which the invention pertains. Generally, the nomenclature used herein and the experiment methods, which will be described below, are those well known and commonly employed in the art.

The present invention provides a double-stranded oligo RNA capable of inhibiting the expression of amphiregulin. Examples of the double-stranded oligo RNA according to the present invention include, but are not limited to, siRNA (short interfering RNA), shRNA (short hairpin RNA) and miRNA (microRNA).

In addition, it is understood that a single-stranded miRNA inhibitor capable of functioning as an antagonist miRNA also falls within the scope of the double-stranded oligo RNA according to the present invention.

Hereinafter, the double-stranded oligo RNA according to the present invention will be described by an example of siRNA. However, it will be obvious to those skilled in the art that the double-stranded oligo RNA according to the present invention is not limited to siRNA and that the same characteristics may also be applied to double-stranded oligo RNAs other than siRNA.

Specifically, the present invention provides an amphiregulin-specific siRNA comprising a first oligonucleotide, which is a sense strand having any one sequence selected from the following SEQ ID NO: 1 to SEQ ID NO: 230, and a second oligonucleotide which is an antisense strand having a sequence complementary to that of the sense strand:
5'-ggaguaugauaaugaacca-3'(SEQ ID NO: 1),
5'-caguaguagcugucacuau-3'(SEQ ID NO: 2),
5'-agacucacagcgaggauga-3'(SEQ ID NO: 3),
5'-cacaaauauccggcuauau-3'(SEQ ID NO: 4),
5'-caccauaagcgaaaugccu-3'(SEQ ID NO: 5),
5'-gauuacuuuggugaacggu-3'(SEQ ID NO: 6),
5'-caguugucacuuuuuauga-3'(SEQ ID NO: 7),
5'-ggaccuauccaagauugca-3'(SEQ ID NO: 8),
5'-cguuaucacagugcaccuu-3'(SEQ ID NO: 9),
5'-ccuagcugaggacaaugca-3'(SEQ ID NO: 10),
5'-ggaagagagguuuccacca-3'(SEQ ID NO: 11),
5'-cucagaggaguaugauaau-3'(SEQ ID NO: 12),
5'-cgguggacuugagcuuucu-3'(SEQ ID NO: 13),
5'-gguggugacaugcaauugu-3'(SEQ ID NO: 14),
5'-cagggaauaugaaggagaa-3'(SEQ ID NO: 15),
5'-ggaggcuucgacaagaaaa-3'(SEQ ID NO: 16),
5'-ccgguggaaccaaugagaa-3'(SEQ ID NO: 17),
5'-ccggcuauauuauagauga-3'(SEQ ID NO: 18),
5'-agaauccaugcacugccaa-3'(SEQ ID NO: 19),
5'-caaggaccuauccaagauu-3'(SEQ ID NO: 20),
5'-caaauauccggcuauauua-3'(SEQ ID NO: 21),
5'-gggacuacgacuacucaga-3'(SEQ ID NO: 22),
5'-gcgaaugcagauacaucga-3'(SEQ ID NO: 23),
5'-gccacaccggaaaugacau-3'(SEQ ID NO: 24),
5'-agaguugaacaggugauua-3'(SEQ ID NO: 25),
5'-gaaccacaaauauccggcu-3'(SEQ ID NO: 26),
5'-cauaagcgaaaugccuucu-3'(SEQ ID NO: 27),
5'-guuuccaccauaagcgaaa-3'(SEQ ID NO: 28),
5'-caggugauuaagcccaaga-3'(SEQ ID NO: 29),
5'-ccacaccggaaaugacauu-3'(SEQ ID NO: 30),
5'-gagugaaaugccuucuagu-3'(SEQ ID NO: 31),
5'-cagaguugaacagguaguu-3'(SEQ ID NO: 32),
5'-cuggauuggaccucaauga-3'(SEQ ID NO: 33),
5'-gaaaacucacagcaugauu-3'(SEQ ID NO: 34),
5'-gaaacuucgacaagagaau-3'(SEQ ID NO: 35),
5'-caggaaauaugaaggagaa-3'(SEQ ID NO: 36),
5'-gcaaauauauagagcaccu-3'(SEQ ID NO: 37),
5'-ggugcugucgcucuugaua-3'(SEQ ID NO: 38),
5'-ucagaguugaacagguagu-3'(SEQ ID NO: 39),
5'-gaaagaaacuucgacaaga-3'(SEQ ID NO: 40),
5'-gacaauacgucaggaaaua-3'(SEQ ID NO: 41),
5'-cggcucaggccauuaugcu-3'(SEQ ID NO: 42),
5'-ggaccacagugcugaugga-3'(SEQ ID NO: 43),
5'-gcugcuggauuggaccuca-3'(SEQ ID NO: 44),
5'-guuauuacaguccagcuua-3'(SEQ ID NO: 45),
5'-uggaccucaaugacaccua-3'(SEQ ID NO: 46),
5'-cuggcuauauugucgauga-3'(SEQ ID NO: 47),
5'-gacggaaagugaaaauacu-3'(SEQ ID NO: 48),
5'-gcagccauagcugccuuua-3'(SEQ ID NO: 49),
5'-ggagccgacuaugacuacu-3'(SEQ ID NO: 50),
5'-guaugauaacgaaccacaa-3'(SEQ ID NO: 51),
5'-agugaaaugccuucuagua-3'(SEQ ID NO: 52),
5'-gauaacgaaccacaaauac-3'(SEQ ID NO: 53),
5'-ugcauuagcagccauagcu-3'(SEQ ID NO: 54),
5'-cgggagccgacuaugacua-3'(SEQ ID NO: 55),
5'-ccgacuaugacuacucaga-3'(SEQ ID NO: 56),
5'-gcauucacggagaaugcaa-3'(SEQ ID NO: 57),
5'-ccaugaaaacucacagcau-3'(SEQ ID NO: 58),
5'-cagcaugauugacaguagu-3'(SEQ ID NO: 59),
5'-cuuagaagacaauacguca-3'(SEQ ID NO: 60),
5'-ugacuacucagaagaguau-3'(SEQ ID NO: 61),
5'-agaguugaacagguaguua-3'(SEQ ID NO: 62),
5'-auauauagagcaccuggaa-3'(SEQ ID NO: 63),
5'-agcagccauagcugccuuu-3'(SEQ ID NO: 64),
5'-gaguugaacagguaguuaa-3'(SEQ ID NO: 65),
5'-cauucacggagaaugcaaa-3'(SEQ ID NO: 66),
5'-ucaggaaauaugaaggaga-3'(SEQ ID NO: 67),
5'-cagaagaguaugauaacga-3'(SEQ ID NO: 68),
5'-gcuguuauuacaguccagc-3'(SEQ ID NO: 69),
5'-gggaagcgugaaccauuuu-3'(SEQ ID NO: 70),
5'-agcugccuuuaugucugcu-3'(SEQ ID NO: 71),
5'-cacagugcugauggauuug-3'(SEQ ID NO: 72),
5'-agucagaguugaacaggua-3'(SEQ ID NO: 73),
5'-uggaagcaguaacaugcaa-3'(SEQ ID NO: 74),
5'-agaagaguaugauaacgaa-3'(SEQ ID NO: 75),
5'-gaagcgugaaccauuuucu-3'(SEQ ID NO: 76),
5'-ggcuauauugucgaugauu-3'(SEQ ID NO: 77),
5'-gacaagagaauggaaaugu-3'(SEQ ID NO: 78),
5'-ugaaaugccuucuaguagu-3'(SEQ ID NO: 79),
5'-gugagugaaaugccuucua-3'(SEQ ID NO: 80),
5'-gaccucaaugacaccuacu-3'(SEQ ID NO: 81),
5'-ccucaaugacaccuacucu-3'(SEQ ID NO: 82),
5'-gcugauggauuugagguua-3'(SEQ ID NO: 83),
5'-ggaagcaguaacaugcaaa-3'(SEQ ID NO: 84),
5'-caguaacaugcaaauguca-3'(SEQ ID NO: 85),
5'-ggaccucaaugacaccuac-3'(SEQ ID NO: 86),
5'-cagccauagcugccuuuau-3'(SEQ ID NO: 87),
5'-uccaugaaaacucacagca-3'(SEQ ID NO: 88),
5'-gcugccuuuaugucugcug-3'(SEQ ID NO: 89),
5'-gcucuugauacucggcuca-3'(SEQ ID NO: 90),
5'-ugcugcuggauuggaccuc-3'(SEQ ID NO: 91),
5'-gaaccacaaauaccuggcu-3'(SEQ ID NO: 92),
5'-agagcaccuggaagcagua-3'(SEQ ID NO: 93),
5'-caccuggaagcaguaacau-3'(SEQ ID NO: 94),
5'-cugaggaacgaaagaaacu-3'(SEQ ID NO: 95),
5'-gugaaaugccuucuaguag-3'(SEQ ID NO: 96),
5'-cuacucugggaagcgugaa-3'(SEQ ID NO: 97),
5'-cugggaagcgugaaccauu-3'(SEQ ID NO: 98),
5'-acuacucagaagaguauga-3'(SEQ ID NO: 99),
5'-caugcaaaugucagcaaga-3'(SEQ ID NO: 100),
5'-ugagguuaccucaagaagu-3'(SEQ ID NO: 101),
5'-acucggcucaggccauuau-3'(SEQ ID NO: 102),
5'-uucacggagaaugcaaaua-3'(SEQ ID NO: 103),
5'-cugcuggauuggaccucaa-3'(SEQ ID NO: 104),
5'-ugauucagucagaguugaa-3'(SEQ ID NO: 105),
5'-ucgacaagagaauggaaau-3'(SEQ ID NO: 106),
5'-cucaagaagugagaugucu-3'(SEQ ID NO: 107),
5'-uucugcauucacggagaau-3'(SEQ ID NO: 108),
5'-agguuaccucaagaaguga-3'(SEQ ID NO: 109),
5'-aaugccuucuaguagugaa-3'(SEQ ID NO: 110),
5'-augauucagucagaguuga-3'(SEQ ID NO: 111),
5'-aaacaagacggaaagugaa-3'(SEQ ID NO: 112),
5'-uuucugcauucacggagaa-3'(SEQ ID NO: 113),
5'-caaauaccuggcuauauug-3'(SEQ ID NO: 114),
5'-gaagcaguaacaugcaaau-3'(SEQ ID NO: 115),
5'-gacuacucagaagaguaug-3'(SEQ ID NO: 116),
5'-gaugauucagucagaguug-3'(SEQ ID NO: 117),
5'-ugcauucacggagaaugca-3'(SEQ ID NO: 118),
5'-gcugaggaacgaaagaaac-3'(SEQ ID NO: 119),
5'-uggauuugagguuaccuca-3'(SEQ ID NO: 120),
5'-agugagaugucuucaggga-3'(SEQ ID NO: 121),
5'-gagguuaccucaagaagug-3'(SEQ ID NO: 122),
5'-cuucgacaagagaauggaa-3'(SEQ ID NO: 123),
5'-cucuugauacucggcucag-3'(SEQ ID NO: 124),
5'-gucagaguugaacagguag-3'(SEQ ID NO: 125),
5'-caugaaaacucacagcaug-3'(SEQ ID NO: 126),
5'-ggaaauguacaugcuauag-3'(SEQ ID NO: 127),
5'-cauguaaugcagaauuuca-3'(SEQ ID NO: 128),
5'-gauugacaguaguuuauca-3'(SEQ ID NO: 129),
5'-guccagcuuagaagacaau-3'(SEQ ID NO: 130),
5'-ugcuggauuggaccucaau-3'(SEQ ID NO: 131),
5'-gaagacaauacgucaggaa-3'(SEQ ID NO: 132),
5'-aagacaauacgucaggaaa-3'(SEQ ID NO: 133),
5'-ugaggaacgaaagaaacuu-3'(SEQ ID NO: 134),
5'-uucgacaagagaauggaaa-3'(SEQ ID NO: 135),
5'-ugcuguuauuacaguccag-3'(SEQ ID NO: 136),
5'-gauggauuugagguuaccu-3'(SEQ ID NO: 137),
5'-cucaaugacaccuacucug-3'(SEQ ID NO: 138),
5'-gguuaccucaagaagugag-3'(SEQ ID NO: 139),
5'-gccgacuaugacuacucag-3'(SEQ ID NO: 140),
5'-caaauauauagagcaccug-3'(SEQ ID NO: 141),
5'-agccgacuaugacuacuca-3'(SEQ ID NO: 142),
5'-accuggcuauauugucgau-3'(SEQ ID NO: 143),
5'-ccuacucugggaagcguga-3'(SEQ ID NO: 144),
5'-gcauuagcagccauagcug-3'(SEQ ID NO: 145),
5'-augauaacgaaccacaaau-3'(SEQ ID NO: 146),
5'-gcgugaaccauuuucuggg-3'(SEQ ID NO: 147),
5'-ugggaagcgugaaccauuu-3'(SEQ ID NO: 148),
5'-agcaguaacaugcaaaugu-3'(SEQ ID NO: 149),
5'-ugaaaacucacagcaugau-3'(SEQ ID NO: 150),
5'-agacaauacgucaggaaau-3'(SEQ ID NO: 151),
5'-auacucggcucaggccauu-3'(SEQ ID NO: 152),
5'-auuaugcugcuggauugga-3'(SEQ ID NO: 153),
5'-ugcugauggauuugagguu-3'(SEQ ID NO: 154),
5'-ugugagugaaaugccuucu-3'(SEQ ID NO: 155),
5'-auucagucagaguugaaca-3'(SEQ ID NO: 156),
5'-aagggaggcaaaaauggaa-3'(SEQ ID NO: 157),
5'-uguuauuacaguccagcuu-3'(SEQ ID NO: 158),
5'-acgaaagaaacuucgacaa-3'(SEQ ID NO: 159),
5'-cugcauucacggagaaugc-3'(SEQ ID NO: 160),
5'-agagaauggaaauguacau-3'(SEQ ID NO: 161),
5'-cgucaggaaauaugaagga-3'(SEQ ID NO: 162),
5'-augcugcuggauuggaccu-3'(SEQ ID NO: 163),
5'-aguaugauaacgaaccaca-3'(SEQ ID NO: 164),
5'-aaccacaaauaccuggcua-3'(SEQ ID NO: 165),
5'-aauauauagagcaccugga-3'(SEQ ID NO: 166),
5'-aaauugcauuagcagccau-3'(SEQ ID NO: 167),
5'-acgaaccacaaauaccugg-3'(SEQ ID NO: 168),
5'-aagcaguaacaugcaaaug-3'(SEQ ID NO: 169),
5'-cgcucuugauacucggcuc-3'(SEQ ID NO: 170),
5'-guaacaugcaaaugucagc-3'(SEQ ID NO: 171),
5'-aauguacaugcuauagcau-3'(SEQ ID NO: 172),
5'-cacggagaaugcaaauaua-3'(SEQ ID NO: 173),
5'-cuguuauuacaguccagcu-3'(SEQ ID NO: 174),
5'-cgacaagagaauggaaaug-3'(SEQ ID NO: 175),
5'-aaaacaagacggaaaguga-3'(SEQ ID NO: 176),
5'-cuauauugucgaugauuca-3'(SEQ ID NO: 177),
5'-aguccaugaaaacucacag-3'(SEQ ID NO: 178),
5'-gcugucgcucuugauacuc-3'(SEQ ID NO: 179),
5'-gcuauauugucgaugauuc-3'(SEQ ID NO: 180),
5'-cgaaagaaacuucgacaag-3'(SEQ ID NO: 181),
5'-agcgugaaccauuuucugg-3'(SEQ ID NO: 182),
5'-augaaaacucacagcauga-3'(SEQ ID NO: 183),
5'-acuucgacaagagaaugga-3'(SEQ ID NO: 184),
5'-caguccagcuuagaagaca-3'(SEQ ID NO: 185),
5'-ugcaaauauauagagcacc-3'(SEQ ID NO: 186),
5'-agaagacaauacgucagga-3'(SEQ ID NO: 187),
5'-gggagccgacuaugacuac-3'(SEQ ID NO: 188),
5'-auggaaauguacaugcuau-3'(SEQ ID NO: 189),
5'-caaaaauugcauuagcagc-3'(SEQ ID NO: 190),
5'-gugcugucgcucuugauac-3'(SEQ ID NO: 191),
5'-ugcugucgcucuugauacu-3'(SEQ ID NO: 192),
5'-uacucggcucaggccauua-3'(SEQ ID NO: 193),
5'-uggcuauauugucgaugau-3'(SEQ ID NO: 194),
5'-auucacggagaaugcaaau-3'(SEQ ID NO: 195),
5'-aaacucacagcaugauuga-3'(SEQ ID NO: 196),
5'-auuacaguccagcuuagaa-3'(SEQ ID NO: 197),
5'-ugauggauuugagguuacc-3'(SEQ ID NO: 198),
5'-ucagaagaguaugauaacg-3'(SEQ ID NO: 199),
5'-agaguaugauaacgaacca-3'(SEQ ID NO: 200),
5'-ugauaacgaaccacaaaua-3'(SEQ ID NO: 201),
5'-acggagaaugcaaauauau-3'(SEQ ID NO: 202),
5'-cuaguagugaaccguccuc-3'(SEQ ID NO: 203),
5'-gaagaguaugauaacgaac-3'(SEQ ID NO: 204),
5'-aaaugccuucuaguaguga-3'(SEQ ID NO: 205),
5'-uaacgaaccacaaauaccu-3'(SEQ ID NO: 206),
5'-auugucgaugauucaguca-3'(SEQ ID NO: 207),
5'-acaugcaaaugucagcaag-3'(SEQ ID NO: 208),
5'-uguugcuguuauuacaguc-3'(SEQ ID NO: 209),
5'-uagagcaccuggaagcagu-3'(SEQ ID NO: 210),
5'-aacgaaagaaacuucgaca-3'(SEQ ID NO: 211),
5'-aaauaccuggcuauauugu-3'(SEQ ID NO: 212),
5'-aauacgucaggaaauauga-3'(SEQ ID NO: 213),
5'-uggaaauguacaugcuaua-3'(SEQ ID NO: 214),
5'-acgucaggaaauaugaagg-3'(SEQ ID NO: 215),
5'-uuggaccucaaugacaccu-3'(SEQ ID NO: 216),
5'-uaacaugcaaaugucagca-3'(SEQ ID NO: 217),
5'-augacuacucagaagagua-3'(SEQ ID NO: 218),
5'-aaauccauguaaugcagaa-3'(SEQ ID NO: 219),
5'-ggaagcgugaaccauuuuc-3'(SEQ ID NO: 220),
5'-ucugggaagcgugaaccau-3'(SEQ ID NO: 221),
5'-uauugucgaugauucaguc-3'(SEQ ID NO: 222),
5'-aguaacaugcaaaugucag-3'(SEQ ID NO: 223),
5'-uuagaagacaauacgucag-3'(SEQ ID NO: 224),
5'-aaaauccauguaaugcaga-3'(SEQ ID NO: 225),
5'-gcuggauuggaccucaaug-3'(SEQ ID NO: 226),
5'-cugauggauuugagguuac-3'(SEQ ID NO: 227),
5'-ccucaagaagugagauguc-3'(SEQ ID NO: 228),
5'-gagccgacuaugacuacuc-3'(SEQ ID NO: 229),
5'-gauucagucagaguugaac-3'(SEQ ID NO: 230)

As used herein, term "amphiregulin-specific siRNA(s)" refers to siRNA(s) specific for a gene that encodes amphiregulin protein. In addition, it will be obvious to those skilled in the art that an amphiregulin-specific siRNA comprising an sense strand and an antisense strand, which comprise a substitution, deletion or insertion of one or more nucleotides in a sense strand having a sequence, selected from among SEQ ID NO: 1 to SEQ ID NO: 230, and an antisense strand having a sequence complementary to that of the sense strand, also falls within the scope of the present invention, as long as it has specificity for amphiregulin.

The amphiregulin-specific siRNA according to the present invention has a nucleotide sequence designed such that it can bind complementary to an mRNA encoding amphiregulin, and thus it can effectively inhibit the expression of amphiregulin. Also, the 3' end of one or both strands of the siRNA may comprise an overhang having one or two or more unpaired nucleotides.

In addition, the siRNA may comprise various chemical modifications for imparting nuclease resistance and reducing non-specific immune responses in order to increase the *in vivo* stability thereof.

A modification of the first or second oligonucleotide of the siRNA according to the present invention may be at least one modification or a combination of two or more modifications selected from among: modification in which an OH group at the 2' carbon position of a sugar structure in one or more nucleotides is substituted with-CH₃ (methyl),-OCH₃ (methoxy), -NH₂, -F (fluorine), -O-2-methoxyethyl-O-propyl, -O-2- methylthioethyl, -O-3-aminopropyl, -O-3-dimethylaminopropyl, -O-N-methylacetamido or -O-dimethylamidooxyethyl; modification in which oxygen in a sugar structure in nucleotides is substituted with sulfur; modification of a bond between nucleotides to a phosphorothioate, boranophosphophate or methyl phosphonate bond; and modification to PNA (peptide nucleic acid), LNA (locked nucleic acid) or UNA (unlocked nucleic acid)(Ann. Rev. Med. 55, 61-65 2004; US 5,660,985; US 5,958,691; US 6,531,584; US 5,808,023; US 6,326,358; US 6,175,001; Bioorg. Med. Chem. Lett. 14:1139-1143, 2003; RNA, 9: 1034-1048, 2003; Nucleic Acid Res. 31:589-595, 2003; Nucleic Acids Research, 38(17) 5761-5773, 2010; Nucleic Acids Research, 39(5) 1823-1832, 2011).

Among the amphiregulin-specific siRNAs according to the present invention, an siRNA comprising a sense strand having a sequence selected from SEQ ID NO: 1 to SEQ ID NO: 30 is specific for mouse amphiregulin, and an siRNA comprising a sense strand having a sequence selected from SEQ ID NO: 31 to SEQ ID NO: 230 is specific for human amphiregulin. In the present invention, it was found that siRNAs comprising sense strands having sequences of SEQ ID NOS: 1 to 230 can all effectively inhibit the expression of amphiregulin.

In another aspect, the present invention provides a double-stranded oligo RNA structure that is an siRNA conjugate in which one or more hydrophilic polymers and hydrophobic polymers are conjugated to both ends of the amphiregulin-specific siRNA, respectively, in order to ensure the efficient *in vivo* delivery of the siRNA and increase the stability of the siRNA.

As used herein, the term "double-stranded oligo RNA structure" refers to a double-stranded oligo RNA structure that comprises one or more hydrophilic polymers and hydrophobic polymers conjugated to both ends of siRNA or shRNA, respectively.

The double-stranded oligo RNA structure as described above forms self-assembled nanoparticles by the hydrophobic interaction of the hydrophobic polymer moieties (see Korean Patent Laid-Open Publication No. 2010-123214). This conjugate has advantages in that it has very high *in vivo* delivery efficiency and *in vivo* stability, and also has a uniform particle size distribution, and thus the quality control (QC) thereof is easy so that a process for preparing a drug from the conjugate is simple.

Specifically, an amphiregulin-specific double-stranded oligo RNA structure according to the present invention preferably has a structure represented by the following structural formula (1):

Structural formula (1) A-X-R-Y-B

wherein A represents a hydrophilic compound, B represents a hydrophobic compound, X and Y each independently represents a simple covalent bond or a linker-mediated covalent bond, and R represents the amphiregulin-specific siRNA.

More preferably, the amphiregulin-specific double-stranded oligo RNA structure according to the present invention has a structure represented by the following structural formula (2): 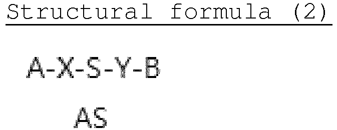 wherein A represents a hydrophilic compound, B represents a hydrophobic compound, X and Y each independently represents a simple covalent bond or a linker-mediated covalent bond, S represents the sense strand of the amphiregulin-specific siRNA, and AS represents the antisense strand of the amphiregulin-specific siRNA.

More preferably, the amphiregulin-specific double-stranded oligo RNA structure comprising the amphiregulin-specific siRNA has a structure represented by the following structural formula (3): 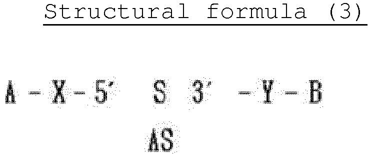 wherein A represents a hydrophilic compound, B represents a hydrophobic compound, X and Y each independently represents a simple covalent bond or a linker-mediated covalent bond, S represents the sense strand of the amphiregulin-specific siRNA, and AS represents the antisense strand of the amphiregulin-specific siRNA.

One to three phosphate groups may be bound to the 5' end of the antisense strand of the double-stranded oligo RNA structure comprising the amphiregulin-specific siRNA as shown in structural formula (1) to structural formula (3). Also, it will be obvious to those skilled in the art that shRNA may be used in place of the siRNA as shown in structural formula (1) to structural formula (3).

The hydrophilic compound in structural formula (1) to structural formula (3) above is preferably a cationic or nonionic polymer compound having a molecular weight of 200-10,000, and more preferably a nonionic polymer compound having a molecular weight of 1,000-2,000. For example, the hydrophilic polymer compound that is used in the present invention is preferably a nonionic hydrophilic polymer compound such as polyethylene glycol, polyvinyl pyrrolidone or polyoxazoline, but is not limited thereto.

The hydrophobic compound (B) in structural formula (1) to structural formula (3) functions to form nanoparticles made of the oligonucleotide structure as shown in structural formula (1) by hydrophobic interactions. The hydrophobic compound preferably has a molecular weight of 250-1,000, and may be a steroid derivative, a glyceride derivative, glycerol ether, polypropylene glycol, a C₁₂-C₅₀ unsaturated or saturated hydrocarbon, diacylphosphatidylcholine, fatty acid, phospholipid, lipopolyamine or the like, but is not limited thereto. It will be obvious to those skilled in the art that any hydrophobic compound may be used, as long as it coincides with the purpose of the present invention.

The steroid derivative may be selected from the group consisting of cholesterol, cholestanol, cholic acid, cholesteryl formate, cholestanyl formate, and cholestanylamine, in which the glyceride derivative may be selected from mono-, di-, and tri-glycerides, and the like. Herein, fatty acid of the glyceride is preferably C₁₂-C₅₀ unsaturated or saturated fatty acid.

Particularly, among the hydrophobic compounds, the saturated or unsaturated hydrocarbon or cholesterol is preferably used, because it can be easily bound in a process of synthesizing the oligonucleotide structure according to the present invention. The hydrophobic compound may be bound to the distal end of the hydrophilic compound, and may be bound to any position of the sense or antisense strand of the siRNA.

The hydrophilic compound or hydrophobic compound in structural formulas (1) to (3) according to the present invention is bound to the amphiregulin-specific siRNA by a single covalent bond or a linker-mediated covalent (X or Y). The linker that mediates the covalent bond is covalently bound to the hydrophilic or hydrophobic compound at the end of the amphiregulin-specific siRNA, and is not specifically limited as long as it provides a degradable bond in a specific environment, if required. Therefore, the linker that is used in the present invention may be any compound that is bound in order to activate the amphiregulin-specific siRNA and/or the hydrophilic (or hydrophobic) compound in the process of preparing the double-stranded oligo RNA structure according to the present invention. The covalent bond may be any one of a non-degradable bond and a degradable bond. Herein, examples of the non-degradable bond include, but are not limited to, an amide bond and a phosphate bond, and examples of the degradable bond include, but are not limited to, a disulfide bond, an acid-degradable bond, an ester bond, an anhydride bond, a biodegradable bond, and an enzyme-degradable bond.

As the amphiregulin-specific siRNA represented by R (or S and AS) in structural formulas (1) to (3), any siRNA having the property of binding specifically to amphiregulin may be used without limitation. Preferably, the siRNA that is used in the present invention comprises a sense strand comprising any one sequence selected from among SEQ ID NO: 1 to SEQ ID NO: 230, and an antisense strand having a sequence complementary to that of the sense strand.

In still another aspect, the present invention provides nanoparticles comprising the amphiregulin-specific double-stranded oligo RNA structure.

As described above, the amphiregulin-specific double-stranded oligo RNA structure comprising the amphiregulin-specific siRNA is an amphiphilic structure comprising both the hydrophobic compound and the hydrophilic compound, in which the hydrophilic moiety is oriented toward of the structure by interactions (such as hydrogen bonds) with water molecules *in vivo,* and the hydrophobic compound moieties are oriented toward the inside of the structure by hydrophobic interactions therebetween to thereby form thermodynamically stable nanoparticles. In other words, the hydrophobic compound is located in the center of the nanoparticles, and the hydrophilic compound is located on the outside of the amphiregulin-specific siRNA to cover the amphiregulin-specific siRNA, thereby forming nanoparticles. The nanoparticles formed as described above enhance the intracellular delivery of the amphiregulin-specific siRNA and increases the efficiency of the amphiregulin-specific siRNA.

The nanoparticles according to the present invention is characterized in that they may be composed only of double-stranded oligo RNA structures comprising siRNAs having the same sequence or may be composed only of double-stranded oligo RNA structures comprising siRNAs having different sequences. In other words, it is understood that the siRNAs may have different sequences while having the same target gene specificity for amphiregulin. Further, the nanoparticles may also include double-stranded oligo RNA structures comprising siRNAs specific for cancer-specific target genes other than amphiregulin.

The amphiregulin-specific siRNA that is included in the double-stranded oligo RNA structures of the nanoparticles according to the present invention preferably comprises a sense strand having any one sequence selected from SEQ ID NOS: 1 to 230, and an antisense strand having a sequence complementary to that of the sense strand, but is not limited thereto, and it will be obvious to those skilled in the art that any siRNA may be used, as long as it has specificity for amphiregulin and coincides with the purpose of the present invention.

The present invention also provides a composition for preventing or treating respiratory diseases, which comprises the amphiregulin-specific siRNA, the double-stranded oligo RNA structure comprising the amphiregulin-specific siRNA, and/or nanoparticles comprising the double-stranded oligo RNA structures.

The composition according to the present invention, which comprises the amphiregulin-specific siRNA, the double-stranded oligo RNA structure comprising the amphiregulin-specific siRNA, and/or nanoparticles comprising the double-stranded oligo RNA structures, exhibits the effect of preventing or treating respiratory diseases by inhibiting pulmonary artery remodeling and airway remodeling. Thus, the composition according to the present invention is effective for the prevention or treatment of respiratory diseases, including asthma, chronic obstructive pulmonary disease (COPD), idiopathic pulmonary fibrosis (IPF), acute/chronic bronchitis, allergic rhinitis, coughing, phlegm, acute lower respiratory tract infections (bronchitis and bronchiolitis), and acute upper respiratory tract infections such as sore throat, tonsillitis or laryngitis.

The composition of the present invention may comprise, in addition to the active ingredient, one or more pharmaceutically acceptable carriers. The pharmaceutically acceptable carriers should be compatible with the active ingredient, and may be one selected from among physiological saline, sterile water, Ringer's solution, buffered saline, dextrose solution, maltodextrin solution, glycerol, ethanol, and a mixture of two or more thereof. If necessary, the composition may contain other conventional additives such as an antioxidant, a buffer or a bacteriostatic agent. In addition, a diluent, a dispersing agent, a surfactant, a binder and a lubricant may additionally be added to the composition to prepare injectable formulations such as an aqueous solution, a suspension and an emulsion. Particularly, the composition is preferably provided as a lyophilized formulation. For the preparation of a lyophilized formulation, a conventional method known in the technical field to which the present invention pertains may be used, and a stabilizer for lyophilization may also be added. Furthermore, the composition can preferably be formulated according to diseases or components by a suitable method known in the art or by a method disclosed in Remington's Pharmaceutical Science, Mack Publishing Company, Easton PA.

The content of the active ingredient in the composition of the present invention and the method for administration of the composition can generally be determined by those skilled in the art based on the condition of the patient and the severity of the disease. In addition, the composition can be formulated in various forms, including powder, tablet, capsule, liquid, injectable solution, ointment and syrup formulations, and may be provided by use of a unit dosage form or multi-dosage container, for example, a sealed ampule or vial.

The composition of the present invention may be administered orally or parenterally. The composition according to the present invention may be administered, for example, orally, intravenously, intramuscularly, intraarterially, intramedullarily, intradually, intracardially, transdermally, subcutaneously, intraperitoneally, intrarectally, sublingually or topically, but is not limited thereto. Particularly, the composition according to the present invention may be administered to the lung through endobronchial instillation for the treatment of respiratory diseases. The dose of the composition according to the present invention may vary depending on the patient's weight, age, sex, health condition and diet, the time of administration, the mode of administration, excretion rate, the severity of the disease, or the like, and can be easily determined by those skilled in the art. In addition, for clinical administration, the composition of the present invention may be prepared into a suitable formulation using a known technique.

In addition, the present invention provides the use of the amphiregulin-specific siRNA according to the present invention, the double-stranded oligo RNA structure comprising the siRNA, or a composition or nanoparticles comprising the same, for the manufacture of a medicament for preventing or treating respiratory diseases. Additionally, the present invention provides a method for preventing or treating respiratory diseases, which comprises administering the double-stranded oligo RNA structure of the present invention or a composition or nanoparticles comprising the RNA structure to a patient in need thereof.

### EXAMPLES

Hereinafter, the present invention will be described in further detail with reference to examples. It will be obvious to a person having ordinary skill in the art that these examples are illustrative purposes only and are not to be construed to limit or change the scope of the present invention.

### Example 1: Design of target nucleotide sequence and preparation of siRNA

Based on the mouse amphiregulin mRNA sequence (NM_009704_ver1), a target nucleotide sequence (sense strand) to which siRNA can bind was designed, and an siRNA comprising an antisense strand having a sequence complementary to the target nucleotide sequence was prepared (Nucleic Acids Research,12:4539-4557, 1984). Specifically, using a gene design program (Turbo si-Designer) developed by Bioneer (Korea), a target nucleotide sequence to which siRNA can bind was designed based on the amphiregulin mRNA sequence (NM_009704). The amphiregulin siRNA according to the present invention has a double-stranded structure comprising a sense strand consisting of 19 nucleotides and an antisense strand complementary to the sense strand. The siRNA was prepared by connecting the phosphodiester bonds of the RNA backbone structure using (β-cyanoethyl phosphoramidite (Nucleic Acids Research,12:4539-4557, 1984). Specifically, a series of processes including deblocking, coupling, oxidation and capping were repeated on a nucleotide-bound solid support using a RNA synthesizer (384 Synthesizer, BIONEER, KOREA), thereby obtaining a reaction product comprising a RNA having a desired length. The RNA was separated and purified from the reaction product by HPLC LC918 (Japan Analytical Industry, Japan) equipped with a Daisogel C18 column (Daiso, Japan), and was analyzed by a MALDI-TOF mass spectrometer to confirm whether it was consistent with the desired nucleotide sequence. Next, the sense and antisense RNA strands were bound to each other, thereby preparing double-stranded siRNAs comprising a sense strand having each of the sequences of SEQ ID NOS: 1 to 30.

### Example 2: Inhibition of expression of amphiregulin in fibroblast (NIH3T3) cell line by siRNA

A mouse fibroblast (NIH3T3) cell line was transformed with the amphiregulin-specific siRNAs (prepared in Example 1) having the sense strands of SEQ ID NO: 1 to SEQ ID NO: 30, respectively, and the expression patterns of amphiregulin in the transformed fibroblast (NIH3T3) cells, that is, the degrees of inhibition of expression of amphiregulin by the siRNAs, were analyzed.

### 2-1: Culture of fibroblast cell line

The mouse fibroblast cell line (NIH3T3) obtained from the American Type Culture Collection (ATCC) was cultured in RPMI-1640 medium (GIBCO/Invitrogen, USA, 10%(v/v) fetal bovine serum, 100 units/mℓ, penicillin and 100 *µ*g/mℓ, streptomycin) under the conditions of 37°C and 5%(v/v) CO₂.

### 2-2: Transfection of target siRNAinto fibroblast cell line

1×10⁵ fibroblast cells cultured in Example 2-1 were cultured in RPMI 1640 in a 12-well plate for 18 hours under the conditions of 37°C and 5%(v/v) CO₂, and then the medium was removed, after which 500 *µ*ℓ of Opti-MEM medium (GIBCO, USA) was dispensed into each well.

Meanwhile, a mixture of 2 *µ*ℓ of Lipofectamine™ 2000 (Invitrogen, USA) and 248 *µ*ℓ of Opti-MEM medium was allowed to react at room temperature for 5 minutes, and 20 *µ*ℓ of the siRNA (1 pmole/*µ*e) comprising a sense strand having a sequence of any one of SEQ ID NOS: 1 to 30, prepared in Example 1, was added to 230 *µ*ℓ of Opti-MEM medium to prepare siRNA solutions having a final concentration of 20 nM. The Lipofectamine 2000 mixture and each of the siRNA solutions were mixed and incubated at room temperature for 20 minutes to prepare transfection solutions.

Next, 500 *µ*ℓ of each of the transfection solutions was dispensed into each well containing the tumor cell line and Opti-MEM and was incubated for 6 hours, after which the Opti-MEM medium was removed. Then, 1 mℓ of RPMI 1640 medium was dispensed into each well, and each well was incubated for 24 hours under the conditions of 37°C and 5%(v/v) CO₂.

### 2-3: Quantitative analysis of amphiregulin mRNA

A total RNA was extracted from the cell line transfected in Example 2-2 and was synthesized into cDNA, and then the expression level of amphiregulin mRNA was relatively quantified by real-time PCR.

### 2-3-1: Isolation of RNA from transfected cells and synthesis of cDNA

A total RNA was extracted from the cell line, transfected in Example 2-2, using an RNA extraction kit (AccuPrep Cell total RNA extraction kit, BIONEER, Korea), and the extracted RNA was synthesized into cDNA using RNA reverse transcriptase (AccuPower CycleScript RT Premix/dT20, Bioneer, Korea) in the following manner. Specifically, 1 *µ*g/tube of the extracted RNA was added to AccuPower CycleScript RT Premix/dT20 (Bioneer, Korea) in a 0.25 mℓ Eppendorf tube, and distilled water treated with DEPC (diethyl pyrocarbonate) was added thereto to a total volume of 20 *µ*ℓ. Then, the RNA and primers were hybridized using a gene amplification system (MyGenie™ 96 Gradient Thermal Block, BIONEER, Korea) at 30°C for 1 minute, and a cDNA was synthesized at 52°C for 4 minutes. The hybridization and cDNA synthesis processes were repeated six times, and then the enzyme was inactivated at 95°C for 5 minutes, thereby amplifying the cDNA.

### 2-3-2: Relative quantitative analysis of amphiregulin mRNA

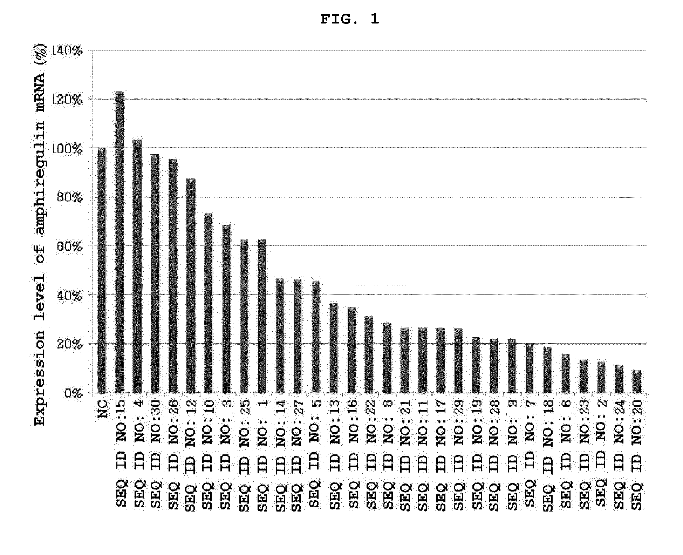

Using the cDNA synthesized in Example 2-3-1 as a template, the relative level of amphiregulin mRNA was quantified by real-time PCR in the following manner. Specifically, the cDNA synthesized in Example 2-3-1 was 5-fold diluted with distilled water, and to analyze the expression level of amphiregulin mRNA, 3 *µ*ℓ of the diluted cDNA, 10 *µ*ℓ of 2×GreenStar™ PCR master mix (BIONEER, Korea), 6 *µ*ℓ of distilled water and 1 *µ*ℓ of amphiregulin qPCR primers (10 pmole/*µ*ℓ for each primer, BIONEER, Korea) were added to each well of a 96-well plate to make a mixture. Meanwhile, GAPDH (glyceraldehyde 3-phosphate dehydrogenase), a housekeeping gene (hereinafter referred to as HK gene), was used as a standard gene to normalize the expression level of amphiregulin mRNA. The 96-well plate containing the mixture was subjected to the following reaction using Exicycler™96 Real-Time Quantitative Thermal Block (BIONEER, Korea). Specifically, the mixture was allowed to react at 95°C for 15 minutes to activate the enzyme and remove the secondary structure of the cDNA, and then the mixture was subjected to 42 cycles, each consisting of denaturation at 94°C for 30 sec, annealing at 58°C for 30 sec, extension at 72°C for 30 sec, and SYBR green scan, and final extension at 72°C for 3 minutes. Next, the mixture was maintained at a temperature of 55°C for 1 minute, and the melting curve from 55°C to 95°C was analyzed. After completion of the PCR amplification, the Ct (threshold cycle) value of amphiregulin was corrected by calculating the mRNA value (normalization factor, NF) normalized by the GAPDH gene, and then the ΔCt value was calculated using a control not treated with the amphiregulin-specific siRNA. The expression level of amphiregulin mRNA caused by the amphiregulin-specific siRNA comprising the sense strand having the sequence of each of SEQ ID NO: 1 to SEQ ID NO: 30 was relatively quantified using the ΔCt value and the equation 2 (^{-ΔCt})×100 (see FIG. 1).

In order to select high-efficiency siRNAs, 15 different siRNAs comprising a sense strand having each of the sequences of SEQ ID NOS: 2, 6 to 9, 11, 17 to 21, 23, 24, 28 and 29 were selected, which showed a decrease in amphiregulin mRNA expression level of 70% or higher compared to the control at a final concentration of 20 nM.

### Example 3: Inhibition of expression of amphiregulin in fibroblast (NIH3T3) cell line by selected siRNAs

A mouse fibroblast (NIH3T3) cell line was transformed with the amphiregulin-specific siRNAs (selected in Example 2) comprising the sense strand having each of the sequences of SEQ ID NOS: 2, 6 to 9, 11, 17 to 21, 23, 24, 28 and 29, and the expression patterns of amphiregulin mRNA in the transformed fibroblast (NIH3T3) cells were analyzed.

### 3-1: Culture of fibroblast cell line

The mouse fibroblast cell line (NIH3T3) obtained from the American Type Culture Collection (ATCC) was cultured in RPMI-1640 medium (GIBCO/Invitrogen, USA, 10%(v/v) fetal bovine serum, 100 units/mℓ, penicillin and 100 *µ*g/mℓ, streptomycin) under the conditions of 37°C and 5%(v/v) CO₂.

### 3-2: Transfection of target siRNAinto fibroblast cell line

1×10⁵ fibroblast cells cultured in Example 2-1 were cultured in RPMI 1640 in a 12-well plate for 18 hours under the conditions of 37°C and 5%(v/v) CO₂, and then the medium was removed, after which 500 *µ*ℓ of Opti-MEM medium (GIBCO, USA) was dispensed into each well.

Meanwhile, a mixture of 2 *µ*ℓ of Lipofectamine 2000 (Invitrogen, USA) and 248 *µ*ℓ of Opti-MEM medium was allowed to react at room temperature for 5 minutes, and 5 *µ*ℓ of the siRNA (1 pmole/*µ*ℓ ) comprising the sense strand having each of the sequences of SEQ ID NOS: 2, 6 to 9, 11, 17 to 21, 23, 24, 28 and 29, selected in Example 2, was added to 245 *µ*ℓ of Opti-MEM medium to prepare siRNA solutions having a final concentration of 20 nM. The Lipofectamine 2000 mixture and each of the siRNA solutions were mixed and incubated at room temperature for 20 minutes to prepare transfection solutions.

Next, 500 *µ*ℓ of each of the transfection solutions was dispensed into each well containing the tumor cell line and Opti-MEM and was incubated for 6 hours, after which the Opti-MEM medium was removed. Then, 1 mℓ of RPMI 1640 medium was dispensed into each well, and each well was incubated for 24 hours under the conditions of 37°C and 5%(v/v) CO₂.

### 3-3: Quantitative analysis of amphiregulin mRNA

A total RNA was extracted from the cell line transfected in Example 3-2 and was synthesized into cDNA, and then the expression level of amphiregulin mRNA in the cells was relatively quantified by real-time PCR.

### 3-3-1: Isolation of RNA from transfected cells and synthesis of cDNA

A total RNA was extracted from the cell line, transfected in Example 2-2, using an RNA extraction kit (AccuPrep Cell total RNA extraction kit, BIONEER, Korea), and the extracted RNA was synthesized into cDNA using RNA reverse transcriptase (AccuPower CycleScript RT Premix/dT20, Bioneer, Korea) in the following manner. Specifically, 1 *µ*g/tube of the extracted RNA was added to AccuPower CycleScript RT Premix/dT20 (Bioneer, Korea) in a 0.25 mℓ Eppendorf tube, and distilled water treated with DEPC (diethyl pyrocarbonate) was added thereto to a total volume of 20 (*µ*ℓ. Then, the RNA and primers were hybridized using a gene amplification system (MyGenie™ 96 Gradient Thermal Block, BIONEER, Korea) at 30°C for 1 minute, and a cDNA was synthesized at 52°C for 4 minutes. The hybridization and cDNA synthesis processes were repeated six times, and then the enzyme was inactivated at 95°C for 5 minutes, thereby amplifying the cDNA.

### 3-3-2: Relative quantitative analysis of amphiregulin mRNA

Using the cDNA synthesized in Example 3-3-1 as a template, the relative level of amphiregulin mRNA was quantified by real-time PCR in the following manner. Specifically, the cDNA synthesized in Example 3-3-1 was 5-fold diluted with distilled water, and in order to analyze the expression level of amphiregulin mRNA, 3 *µ*ℓ of the diluted cDNA, 10 *µ*ℓ of 2×GreenStar™ PCR master mix (Bioneer, Korea), 6 *µ*ℓ of distilled water and 1 *µ*ℓ of amphiregulin qPCR primers (each 10pmole/*µ*ℓ, BIONEER, Korea) were added to each well of a 96-well plate to make a mixture. Meanwhile, GAPDH (glyceraldehyde 3-phosphate dehydrogenase), a housekeeping gene (hereinafter referred to as HK gene), was used as a standard gene to normalize the expression level of amphiregulin mRNA. The 96-well plate containing the mixture was subjected to the following reaction using Exicycler™96 Real-Time Quantitative Thermal Block (Bioneer, Korea). Specifically, the mixture was allowed to react at 95°C for 15 minutes to activate the enzyme and remove the secondary structure of the cDNA, and then the mixture was subjected to 42 cycles, each consisting of denaturation at 94°C for 30 sec, annealing at 58°C for 30 sec, extension at 72°C for 30 sec, and SYBR green scan, followed by final extension at 72°C for 3 minutes. Next, the mixture was maintained at a temperature of 55°C for 1 minute, and the melting curve from 55°C to 95°C was analyzed. After completion of the PCR amplification, the Ct (threshold cycle) value of amphiregulin was corrected by calculating the mRNA value (normalization factor, NF) normalized by the GAPDH gene, and then the ΔCt value was calculated using a control not treated with the amphiregulin-specific siRNA. The change in expression level of amphiregulin mRNA caused by the amphiregulin-specific siRNA comprising the sense strand having each of the sequences of SEQ ID NOS: 2, 6 to 9, 11, 17 to 21, 23, 24, 28 and 29 was relatively quantified using the ΔCt value and the equation 2(^{-ΔCt})×100.

In order to select high-efficiency siRNAs, three siRNAs comprising sense strands having sequences of SEQ ID NOS: 8, 9 and 28, respectively, were selected, which show the greatest decrease in the amphiregulin mRNA expression level as compared to the control at a final concentration of 5 nM. The IC₅₀ values of the selected siRNAs were examined to determine the concentration at which the expression of the target gene is inhibited by 50% after treatment with the siRNAs.

### Example 4: Inhibition of expression of amphiregulin in fibroblast (NIH3T3) cell line by selected siRNAs

Mouse fibroblasts (NIH3T3) were transformed with the amphiregulin-specific siRNAs comprising the sense strand having each of the sequences of SEQ ID NOS: 8, 9 and 28, selected in Example 3, and the expression patterns of amphiregulin mRNA in the transformed fibroblast (NIH3T3) cells were analyzed to determine the IC₅₀ values of the siRNAs.

### 4-1: Culture of fibroblast cell line

The mouse fibroblast cell line (NIH3T3) obtained from the American Type Culture Collection (ATCC) was cultured in RPMI-1640 medium (GIBCO/Invitrogen, USA, 10%(v/v) fetal bovine serum, 100 units/mℓ, penicillin and 100 *µ*g/mℓ, streptomycin) under the conditions of 37°C and 5%(v/v) CO₂.

### 4-2: Transfection of target siRNAinto fibroblast cell line

1×10⁵ fibroblast cells cultured in Example 2-1 were cultured in RPMI 1640 in a 12-well plate for 18 hours under the conditions of 37°C and 5%(v/v) CO₂, and then the medium was removed, after which 500 *µ*ℓ of Opti-MEM medium (GIBCO, USA) was dispensed into each well.

Meanwhile, a mixture of 2 *µ*ℓ of Lipofectamine 2000 (Invitrogen, USA) and 248 *µ*ℓ of Opti-MEM medium was allowed to react at room temperature for 5 minutes, and 0.2, 1 and 5 *µ*ℓ of the siRNA (1 pmole/*µ*ℓ) comprising the sense strand having each of the sequences of SEQ ID NOS: 8, 9 and 28, selected in Example 3, were added to 249.8, 249 and 245 *µ*ℓ of Opti-MEM medium to prepare siRNA solutions having final concentrations of 0.2, 1 and 5 nM. The Lipofectamine 2000 mixture and each of the siRNA solutions were mixed and incubated at room temperature for 20 minutes to prepare transfection solutions.

Next, 500 *µ*ℓ of each of the transfection solutions was dispensed into each well containing the tumor cell line and Opti-MEM and was incubated for 6 hours, after which the Opti-MEM medium was removed. Then, 1 mℓ of RPMI 1640 medium was dispensed into each well, and each well was incubated for 24 hours under the conditions of 37°C and 5%(v/v) CO₂.

### 4-3: Quantitative analysis of amphiregulin mRNA

A total RNA was extracted from the cell line transfected in Example 4-2 and was synthesized into cDNA, and then the expression level of amphiregulin mRNA was relatively quantified by real-time PCR.

### 4-3-1: Isolation of RNA from transfected cells and synthesis of cDNA

A total RNA was extracted from the cell line, transfected in Example 2-2, using an RNA extraction kit (AccuPrep Cell total RNA extraction kit, BIONEER, Korea), and the extracted RNA was synthesized into cDNA using RNA reverse transcriptase (AccuPower CycleScript RT Premix/dT20, Bioneer, Korea)in the following manner. Specifically, 1 *µ*g/tube of the extracted RNA was added to AccuPower CycleScript RT Premix/dT20 (Bioneer, Korea) in a 0.25 mℓ Eppendorf tube, and distilled water treated with DEPC (diethyl pyrocarbonate) was added thereto to a total volume of 20 *µ*ℓ*.* Then, the RNA and primers were hybridized using a gene amplification system (MyGenie™ 96 Gradient Thermal Block, BIONEER, Korea) at 30°C for 1 minute, and cDNA was synthesized at 52°C for 4 minutes. The hybridization and cDNA synthesis processes were repeated six times, and then the enzyme was inactivated at 95°C for 5 minutes, thereby amplifying the cDNA.

### 4-3-2: Relative quantitative analysis of amphiregulin mRNA

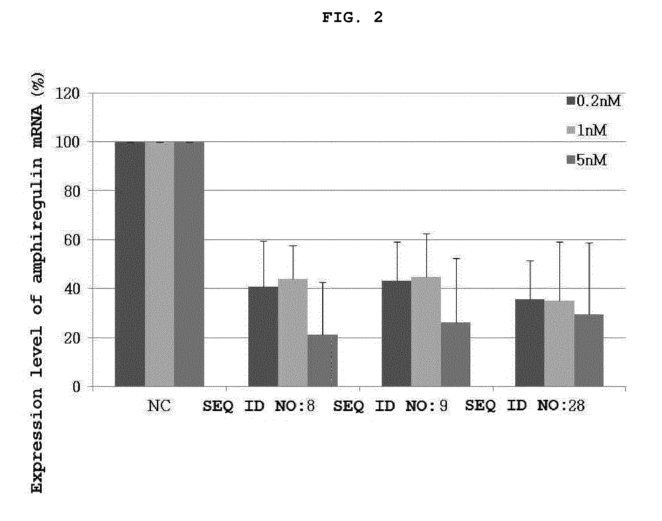

Using the cDNA synthesized in Example 4-3-1 as a template, the relative level of amphiregulin mRNA was quantified by real-time PCR in the following manner. Specifically, the cDNA synthesized in Example 4-3-1 was 5-fold diluted with distilled water, and in order to analyze the expression level of amphiregulin mRNA, 3 *µ*ℓ of the diluted cDNA, 10 *µ*ℓ of 2×GreenStar™ PCR master mix (BIONEER, Korea), 6 *µ*ℓ of distilled water and 1 *µ*ℓ of amphiregulin qPCR primers (each 10pmole/*µ*ℓ, BIONEER, Korea) were added to each well of a 96-well plate to make a mixture. Meanwhile, GAPDH (glyceraldehyde 3-phosphate dehydrogenase), a housekeeping gene (hereinafter referred to as HK gene), was used as a standard gene to normalize the expression level of amphiregulin mRNA. The 96-well plate containing the mixture was subjected to the following reaction using Exicycler™96 Real-Time Quantitative Thermal Block (BIONEER, Korea). Specifically, the mixture was allowed to react at 95°C for 15 minutes to activate the enzyme and remove the secondary structure of the cDNA, and then the mixture was subjected to 42 cycles, each consisting of denaturation at 94°C for 30 sec, annealing at 58°C for 30 sec, extension at 72°C for 30 sec, and SYBR green scan, followed by final extension at 72°C for 3 minutes. Next, the mixture was maintained at a temperature of 55°C for 1 minute, and the melting curve from 55°C to 95°C was analyzed. After completion of the PCR amplification, the Ct (threshold cycle) value of amphiregulin was corrected by calculating the mRNA value (normalization factor, NF) normalized by the GAPDH gene, and then the ΔCt value was calculated using a control not treated with the amphiregulin-specific siRNA. The change in expression level of amphiregulin mRNA caused by each of the amphiregulin-specific siRNAs was relatively quantified using the ΔCt value and the equation 2(^{-ΔCt})×100 (see FIG. 2).

As a result, it was found that the amphiregulin-specific siRNAs having the sense strands of SEQ ID NOS: 8, 9 and 28, respectively, all showed a decrease in amphiregulin mRNA expression level of 50% or higher even at a low concentration of 0.2 nM, suggesting that these siRNAs exhibit the effect of inhibiting the expression of amphiregulin with very high efficiency.

### Example 5: Design of target nucleotide sequence and preparation of siRNA

Based on the human amphiregulin mRNA sequence (NM_001657), a target nucleotide sequence (sense strand) to which an siRNA can bind was designed, and an siRNA comprising an antisense strand having a sequence complementary to the target nucleotide sequence was prepared. Specifically, using a gene design program (Turbo si-Designer) developed by Bioneer (Korea), a target nucleotide sequence to which siRNA can bind was designed based on the amphiregulin mRNA sequence (NM_001657_ver1). The amphiregulin siRNA according to the present invention has a double-stranded structure comprising a sense strand consisting of 19 nucleotides and an antisense strand complementary to the sense strand. In addition, an siCONT (having 5'-CUUACGCUGAGUACUUCGA-3' as a sense strand), an siRNA that does not inhibit the expression of any gene, was prepared. The siRNA was prepared by connecting the phosphodiester bonds of the RNA backbone structure using β-cyanoethyl phosphoramidite (Nucleic Acids Research,12:4539-4557, 1984). Specifically, a series of processes including deblocking, coupling, oxidation and capping were repeated on a nucleotide-bound solid support using a RNA synthesizer (384 Synthesizer, BIONEER, KOREA), thereby obtaining a reaction product comprising a RNA having a desired length. The RNA was separated and purified from the reaction product by HPLC LC918 (Japan Analytical Industry, Japan) equipped with a Daisogel C18 column, and was analyzed by a MALDI-TOF mass spectrometer (Shimadzu, Japan) to confirm whether it was consistent with the desired nucleotide sequence. Next, the sense and antisense RNA strands were bound to each other, thereby preparing siRNAs comprising a sense strand having a sequence of each of SEQ ID NOS: 31 to 230, and an siCONT.

### Example 6: Inhibition of expression of amphiregulin in human lung cancer cell line by siRNA

A human lung cancer cell line (A549) was transformed with each of the siRNAs (prepared in Example 1) having the sense strands of SEQ ID NO: 31 to SEQ ID NO: 230, respectively, and the expression patterns of amphiregulin in the transformed cells, caused by treatment with the siRNAs, were analyzed.

### 6-1: Culture of human lung cancer cell line

The human lung cancer (A549) cell line obtained from the American Type Culture Collection (ATCC) was cultured in RPMI-1640 medium (GIBCO/Invitrogen, USA, 10%(v/v) fetal bovine serum, 100 units/mℓ, penicillin and 100 *µ*g/mℓ, streptomycin) under the conditions of 37°C and 5%(v/v) CO₂.

### 6-2: Transfection of target siRNA into human lung cancer cell line

1×10⁵ human lung cancer (A549) cells cultured in Example 6-1 were cultured in DMEM in a 6-well plate for 18 hours under the conditions of 37°C and 5%(v/v) CO₂, and then the medium was removed, after which 500 *µ*ℓ of Opti-MEM medium (GIBCO, USA) was dispensed into each well.

Meanwhile, a mixture of 3.5 *µ*ℓ of Lipofectamine™ RNAi Max (Invitrogen, USA) and 246.5 *µ*ℓ of Opti-MEM medium was allowed to react at room temperature for 5 minutes, and 5 or 20 *µ*ℓ of the siRNA (1 pmole/*µ*ℓ) comprising a sense strand having a sequence of any one of SEQ ID NOS: 31 to 50, prepared in Example 5, was added to 230 *µ*ℓ of Opti-MEM medium to prepare siRNA solutions having a final concentration of 20 nM. The Lipofectamine max mixture and each of the siRNA solutions were mixed and allowed to react at room temperature for 15 minutes to prepare transfection solutions.

Next, 500 *µ*ℓ of each of the transfection solutions was dispensed into each well containing the tumor cell line and Opti-MEM and was incubated for 6 hours, after which the Opti-MEM medium was removed. Then, 1 *mℓ* of RPMI 1640 medium was dispensed into each well, and each well was incubated for 24 hours under the conditions of 37°C and 5%(v/v) CO₂.

### 6-3: Quantitative analysis of amphiregulin mRNA

A total RNA was extracted from the cell line transfected in Example 6-2 in the same manner as described in Example 2-3-1 and was synthesized into cDNA, and then the mRNA expression level of amphiregulin mRNA was relatively quantified by real-time PCR.

### 6-3-1: Relative quantitative analysis of amphiregulin mRNA

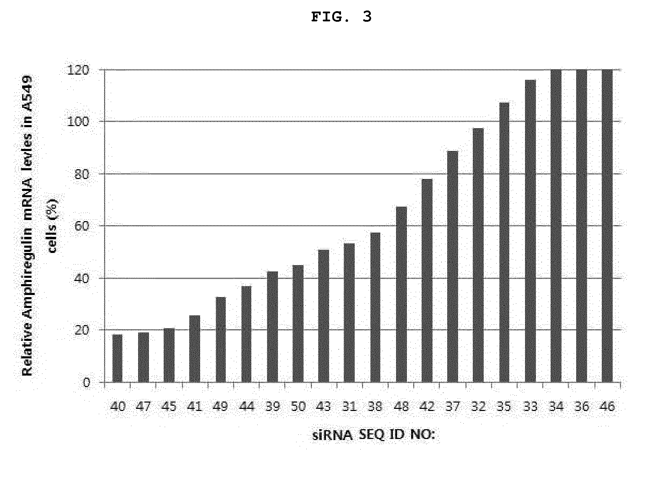

Using the cDNA, prepared in the same manner as Example 2-3-1 from the cell line transfected in Example 6-2, as a template, the relative level of amphiregulin mRNA was quantified by real-time PCR in the following manner. Specifically, the prepared cDNA was 5-fold diluted with distilled water, and to analyze the expression level of amphiregulin mRNA, 3 *µ*ℓ of the diluted cDNA, 10 *µ*ℓ of 2×GreenStar™ PCR master mix (Bioneer, Korea), 6 *µ*ℓ of distilled water and 1 *µ*ℓ of amphiregulin qPCR primers (hAREG-F, ACACCTACTCTGGGAAGCGT; hAREG-R, GCCAGGTATTTGTGGTTCGT; 10 pmole/*µ*ℓ for each primer, Bioneer, Korea) were added to each well of a 96-well plate to make a mixture. Meanwhile, GAPDH (glyceraldehyde 3-phosphate dehydrogenase; hGAPDH-F, GGTGAAGGTCGGAGTCAACG; hGAPDH-R, ACCATGTAGTTGAGGTCAATGAAGG), a housekeeping gene (hereinafter referred to as HK gene), was used as a standard gene to normalize the expression level of amphiregulin mRNA. The 96-well plate containing the mixture was subjected to the following reaction using Exicycler™96 Real-Time Quantitative Thermal Block (Bioneer, Korea). Specifically, the mixture was allowed to react at 95°Cfor 15 minutes to activate the enzyme and remove the secondary structure of the cDNA, and then the mixture was subjected to 42 cycles, each consisting of denaturation at 94°Cfor 30 sec, annealing at 58°Cfor 30 sec, extension at 72°Cfor 30 sec, and SYBR green scan, followed by final extension at 72°Cfor 3 minutes. Next, the mixture was maintained at a temperature of 55°Cfor 1 minute, and the melting curve from 55°Cto 95°Cwas analyzed. After completion of the PCR amplification, the Ct (threshold cycle) value of amphiregulin was corrected by calculating the mRNA value (normalization factor, NF) normalized by the GAPDH gene, and then the ΔCt value was calculated using a control not treated with the amphiregulin-specific siRNA. The expression level of amphiregulin mRNA caused by the amphiregulin-specific siRNA comprising the sense strand having the sequence of each of SEQ ID NO: 31 to SEQ ID NO: 50 was relatively quantified using the ΔCt value and the equation 2(^{-ΔCt})×100 (see FIG. 3). It could be seen that almost all of the amphiregulin-specific siRNAs inhibited the expression of amphiregulin mRNA, and the expression level of amphiregulin mRNA in the cells treated with the amphiregulin-specific siRNAs greatly decreased compared to the control. Among the amphiregulin-specific siRNAs, an siRNA comprising a sense strand having any one sequence selected from SEQ ID NOS: 39 to 41, 43 to 45, 47, 49 and 50 exhibited the highest ability to inhibit the expression of amphiregulin mRNA.

In addition, it was found that an siRNA comprising a sense strand having a sequence of each of SEQ ID NOS: 51 to 230 could inhibit the expression of amphiregulin with very high efficiency, similar to the siRNAs comprising the sense strand having each of the sequences of SEQ ID NOS: 31 to 50.

### INDUSTRIAL APPLICABILITY

As described above, the amphiregulin-specific double-stranded oligo siRNA according to the present invention can very efficiently inhibit the expression of amphiregulin. Thus, a composition comprising the amphiregulin-specific double-stranded oligo siRNA according to the present invention, a double-stranded oligo structure comprising the double-stranded oligo siRNA, and/or nanoparticles composed of the double-stranded oligo structures, can be effectively used for the prevention or treatment of respiratory diseases.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. An amphiregulin-specific siRNA comprising a sense strand comprising any one sequence selected from SEQ ID NO: 1 to SEQ ID NO: 230, and an antisense strand comprising a sequence complementary to that of the sense strand.

2. The amphiregulin-specific siRNA of claim 1, wherein the sense strand or the antisense strand of the siRNA consists of 19-31 nucleotides.

3. The amphiregulin-specific siRNA of claim 1, wherein the sense strand comprising any one sequence selected from the group consisting of SEQ ID NOs: 8, 9, 28, 39 to 41, 43 to 45, 47, 49 and 50, and an antisense strand comprising a sequence complementary to that of the sense strand.

4. The amphiregulin-specific siRNA of any one of claims 1 to 3, wherein the sense strand or the antisense strand of the siRNA comprises at least one chemical modification.

5. The amphiregulin-specific siRNA of claim 4, wherein the chemical modification is at least one modification selected from among: modification in which an OH group at the 2' carbon position of a sugar structure in one or more nucleotides is substituted with -CH₃ (methyl), -OCH₃ (methoxy), -NH₂, -F (fluorine), -O-2-methoxyethyl-O-propyl, -O-2-methylthioethyl, -O-3-aminopropyl, -O-3-dimethylaminopropyl, -O-N-methylacetamido or -O-dimethylamidooxyethyl; modification in which oxygen in a sugar structure in nucleotides is substituted with sulfur; modification of a bond between nucleotides to a phosphorothioate, boranophosphophate or methyl phosphonate bond; and modification to PNA (peptide nucleic acid), LNA (locked nucleic acid) or UNA (unlocked nucleic acid).

6. The amphiregulin-specific siRNA of any one of claims 1 to 5, wherein one or more phosphate groups are bound to the 5' end of the antisense strand of the amphiregulin-specific siRNA.

7. A double-stranded oligo RNA structure comprising a structure represented by the following structural formula (1) :
Structural formula (1) A-X-R-Y-B
wherein A represents a hydrophilic compound, B represents a hydrophobic compound, X and Y each independently represents a simple covalent bond or a linker-mediated covalent bond, and R represents the amphiregulin-specific siRNA.

8. The double-stranded oligo RNA structure of claim 7, which has a structure represented by the following structural formula (2): 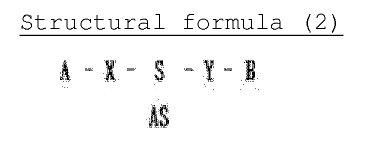 wherein S and AS are the sense strand and the antisense strand of the amphiregulin-specific siRNA, respectively, and A, B, X and Y are the same as defined in claim 7.

9. The double-stranded oligo RNA structure of claim 8, which has a structure represented by the following structural formula (3): 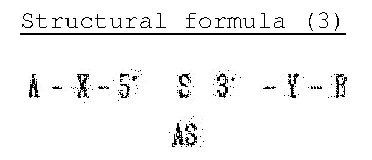 wherein A, B, X, Y, S and AS are the same as defined in claim 8, and 5' and 3' represent the 5' end and the 3' end of the sense strand of the amphiregulin-specific siRNA, respectively.

10. The double-stranded oligo RNA structure of any one of claims 7 to 9, wherein the amphiregulin-specific siRNA is the siRNA of any one of claims 1 to 6.

11. The double-stranded oligo RNA structure of any one of claims 7 to 10, wherein the hydrophilic compound has a molecular weight of 200-10,000.

12. The double-stranded oligo RNA structure of claims 11, wherein the hydrophilic compound is any one selected from the group consisting of polyethylene glycol, polyvinyl pyrrolidone, and polyoxazoline.

13. The double-stranded oligo RNA structure of any one of claims 7 to 10, wherein the hydrophobic compound has a molecular weight of 250-10,000.

14. The double-stranded oligo RNA structure of claim 13, wherein the hydrophobic compound is selected from group consisting of a steroid derivative, a glyceride derivative, glycerol ether, polypropylene glycol, a C₁₂-C₅₀ unsaturated or saturated hydrocarbon, diacylphosphatidylcholine, fatty acid, phospholipid, and lipopolyamine.

15. The double-stranded oligo RNA structure of claim 14 to 14, wherein the steroid derivative is selected from the group consisting of cholesterol, cholestanol, cholic acid, cholesteryl formate, cholestanyl formate, and cholestanylamine.

16. The double-stranded oligo RNA structure of claim 14, wherein the glyceride derivative is selected from mono-, di-, and tri-glycerides.

17. The double-stranded oligo RNA structure of any one of claims 7 to 16, wherein the covalent bond represented by X and Y is either a non-degradable bond or a degradable bond.

18. The double-stranded oligo RNA structure of claim 17, wherein the non-degradable bond is either an amide bond or a phosphate bond.

19. The double-stranded oligo RNA structure of claim 17, wherein the degradable bond is a disulfide bond, an acid-degradable bond, an ester bond, an anhydride bond, a biodegradable bond, or an enzyme-degradable bond.

20. Nanoparticles comprising the double-stranded oligo RNA structure of any one of claims 7 to 19.

21. The Nanoparticles of claim 20, which are composed of double-stranded oligo RNA structures comprising siRNAs comprising different sequences.

22. A pharmaceutical composition which comprises, as an active ingredient, the amphiregulin-specific double-stranded oligo RNA of any one of claims 1 to 6, the double-stranded oligo RNA structure of any one of claims 7 to 19, or the nanoparticles of claim 20 or 21.

23. The pharmaceutical composition of claim 22, which is used for preventing or treating respiratory diseases.

24. The pharmaceutical composition of claim 23, wherein the respiratory diseases are selected from among chronic obstructive pulmonary disease (COPD), idiopathic pulmonary fibrosis (IPF), acute/chronic bronchitis, allergic rhinitis, coughing, phlegm, bronchitis, bronchiolitis, sore throat, tonsillitis, and laryngitis.

25. A lyophilized formulation comprising the pharmaceutical composition of any one of claims 22 to 24.

26. A method for preventing or treating respiratory diseases, which comprises administering, the amphiregulin-specific double-stranded oligo RNA of any one of claims 1 to 6, the double-stranded oligo RNA structure of any one of claims 7 to 19, the nanoparticles of claim 20 or 21, or the composition or the formulation of any one of claims 22 to 25, to a subject in need thereof.

27. The method of claim 26, wherein the respiratory diseases are selected from among chronic obstructive pulmonary disease (COPD), idiopathic pulmonary fibrosis (IPF), acute/chronic bronchitis, allergic rhinitis, coughing, phlegm, bronchitis, bronchiolitis, sore throat, tonsillitis, and laryngitis.


<110>    BIONEER CORPORATION

<120>    Amphiregulin-specific double stranded oligo-RNA, double-stranded
         oligo RNA molecules comprising the double strand oligo-RNA, and
         composition for the prevention or treatment of respiratory
         diseases comprising the same

<130>    PP-B1262

<150>    KR10-2012-0110559
<151>    2012-10-05

<160>    230

<170>    KopatentIn 2.0

<210>    1
<211>    19
<212>    RNA
<213>    Artificial Sequence

<220>
<223>    amphiregulin-specific siRNA


<400>    1
ggaguaugau aaugaacca                                                    19


<210>    2
<211>    19
<212>    RNA
<213>    Artificial Sequence

<220>
<223>    amphiregulin-specific siRNA


<400>    2
caguaguagc ugucacuau                                                    19


<210>    3
<211>    19
<212>    RNA
<213>    Artificial Sequence

<220>
<223>    amphiregulin-specific siRNA


<400>    3
agacucacag cgaggauga                                                    19


<210>    4
<211>    19
<212>    RNA
<213>    Artificial Sequence

<220>
<223>    amphiregulin-specific siRNA

<400>    4
cacaaauauc cggcuauau                                                    19

<210>    5
<211>    19
<212>    RNA
<213>    Artificial Sequence

<220>
<223>    amphiregulin-specific siRNA

<400>    5
caccauaagc gaaaugccu                                                    19

<210>    6
<211>    19
<212>    RNA
<213>    Artificial Sequence

<220>
<223>    amphiregulin-specific siRNA

<400>    6
gauuacuuug gugaacggu                                                    19

<210>    7
<211>    19
<212>    RNA
<213>    Artificial Sequence

<220>
<223>    amphiregulin-specific siRNA

<400>    7
caguugucac uuuuuauga                                                    19

<210>    8
<211>    19
<212>    RNA
<213>    Artificial Sequence

<220>
<223>    amphiregulin-specific siRNA

<400>    8
ggaccuaucc aagauugca                                                    19

<210>    9
<211>    19
<212>    RNA
<213>    Artificial Sequence

<220>

<223>    amphiregulin-specific siRNA


<400>    9
cguuaucaca gugcaccuu                                                    19


<210>    10
<211>    19
<212>    RNA
<213>    Artificial Sequence

<220>
<223>    amphiregulin-specific siRNA


<400>    10
ccuagcugag gacaaugca                                                    19


<210>    11
<211>    19
<212>    RNA
<213>    Artificial Sequence

<220>
<223>    amphiregulin-specific siRNA


<400>    11
ggaagagagg uuuccacca                                                    19


<210>    12
<211>    19
<212>    RNA
<213>    Artificial Sequence

<220>
<223>    amphiregulin-specific siRNA


<400>    12
cucagaggag uaugauaau                                                    19


<210>    13
<211>    19
<212>    RNA
<213>    Artificial Sequence

<220>
<223>    amphiregulin-specific siRNA


<400>    13
cgguggacuu gagcuuucu                                                    19


<210>    14
<211>    19
<212>    RNA
<213>    Artificial Sequence

<220>
<223>    amphiregulin-specific siRNA


<400>    14
gguggugaca ugcaauugu                                                    19


<210>    15
<211>    19
<212>    RNA
<213>    Artificial Sequence

<220>
<223>    amphiregulin-specific siRNA


<400>    15
cagggaauau gaaggagaa                                                    19


<210>    16
<211>    19
<212>    RNA
<213>    Artificial Sequence

<220>
<223>    amphiregulin-specific siRNA


<400>    16
ggaggcuucg acaagaaaa                                                    19


<210>    17
<211>    19
<212>    RNA
<213>    Artificial Sequence

<220>
<223>    amphiregulin-specific siRNA


<400>    17
ccgguggaac caaugagaa                                                    19


<210>    18
<211>    19
<212>    RNA
<213>    Artificial Sequence

<220>
<223>    amphiregulin-specific siRNA


<400>    18
ccggcuauau uauagauga                                                    19


<210>    19
<211>    19

<212>    RNA
<213>    Artificial Sequence

<220>
<223>    amphiregulin-specific siRNA


<400>    19
agaauccaug cacugccaa                                                    19


<210>    20
<211>    19
<212>    RNA
<213>    Artificial Sequence

<220>
<223>    amphiregulin-specific siRNA


<400>    20
caaggaccua uccaagauu                                                    19


<210>    21
<211>    19
<212>    RNA
<213>    Artificial Sequence

<220>
<223>    amphiregulin-specific siRNA


<400>    21
caaauauccg gcuauauua                                                    19


<210>    22
<211>    19
<212>    RNA
<213>    Artificial Sequence

<220>
<223>    amphiregulin-specific siRNA


<400>    22
gggacuacga cuacucaga                                                    19


<210>    23
<211>    19
<212>    RNA
<213>    Artificial Sequence

<220>
<223>    amphiregulin-specific siRNA


<400>    23
gcgaaugcag auacaucga                                                    19

<210>	24
<211>	19
<212>	RNA
<213>	Artificial Sequence

<220>
<223>	amphiregulin-specific siRNA


<400>	24
gccacaccgg aaaugacau	19


<210>	25
<211>	19
<212>	RNA
<213>	Artificial Sequence

<220>
<223>	amphiregulin-specific siRNA


<400>	25
agaguugaac aggugauua	19


<210>	26
<211>	19
<212>	RNA
<213>	Artificial Sequence

<220>
<223>	amphiregulin-specific siRNA


<400>	26
gaaccacaaa uauccggcu	19


<210>	27
<211>	19
<212>	RNA
<213>	Artificial Sequence

<220>
<223>	amphiregulin-specific siRNA


<400>	27
cauaagcgaa augccuucu	19


<210>	28
<211>	19
<212>	RNA
<213>	Artificial Sequence

<220>
<223>	amphiregulin-specific siRNA


<400>	28
guuuccacca uaagcgaaa	19

<210>    29
<211>    19
<212>    RNA
<213>    Artificial Sequence

<220>
<223>    amphiregulin-specific siRNA


<400>    29
caggugauua agcccaaga                                                    19


<210>    30
<211>    19
<212>    RNA
<213>    Artificial Sequence

<220>
<223>    amphiregulin-specific siRNA


<400>    30
ccacaccgga aaugacauu                                                    19


<210>    31
<211>    19
<212>    RNA
<213>    Artificial Sequence

<220>
<223>    amphiregulin-specific siRNA


<400>    31
gagugaaaug ccuucuagu                                                    19


<210>    32
<211>    19
<212>    RNA
<213>    Artificial Sequence

<220>
<223>    amphiregulin-specific siRNA


<400>    32
cagaguugaa cagguaguu                                                    19


<210>    33
<211>    19
<212>    RNA
<213>    Artificial Sequence

<220>
<223>    amphiregulin-specific siRNA

<400>    33
cuggauugga ccucaauga                                                    19


<210>    34
<211>    19
<212>    RNA
<213>    Artificial Sequence

<220>
<223>    amphiregulin-specific siRNA


<400>    34
gaaaacucac agcaugauu                                                    19


<210>    35
<211>    19
<212>    RNA
<213>    Artificial Sequence

<220>
<223>    amphiregulin-specific siRNA


<400>    35
gaaacuucga caagagaau                                                    19


<210>    36
<211>    19
<212>    RNA
<213>    Artificial Sequence

<220>
<223>    amphiregulin-specific siRNA


<400>    36
caggaaauau gaaggagaa                                                    19


<210>    37
<211>    19
<212>    RNA
<213>    Artificial Sequence

<220>
<223>    amphiregulin-specific siRNA


<400>    37
gcaaauauau agagcaccu                                                    19


<210>    38
<211>    19
<212>    RNA
<213>    Artificial Sequence

<220>
<223>    amphiregulin-specific siRNA

<400>    38
ggugcugucg cucuugaua                                                    19


<210>    39
<211>    19
<212>    RNA
<213>    Artificial Sequence

<220>
<223>    amphiregulin-specific siRNA


<400>    39
ucagaguuga acagguagu                                                    19


<210>    40
<211>    19
<212>    RNA
<213>    Artificial Sequence

<220>
<223>    amphiregulin-specific siRNA


<400>    40
gaaagaaacu ucgacaaga                                                    19


<210>    41
<211>    19
<212>    RNA
<213>    Artificial Sequence

<220>
<223>    amphiregulin-specific siRNA


<400>    41
gacaauacgu caggaaaua                                                    19


<210>    42
<211>    19
<212>    RNA
<213>    Artificial Sequence

<220>
<223>    amphiregulin-specific siRNA


<400>    42
cggcucaggc cauuaugcu                                                    19


<210>    43
<211>    19
<212>    RNA
<213>    Artificial Sequence

<220>
<223>    amphiregulin-specific siRNA


<400>    43
ggaccacagu gcugaugga                                                    19


<210>    44
<211>    19
<212>    RNA
<213>    Artificial Sequence

<220>
<223>    amphiregulin-specific siRNA


<400>    44
gcugcuggau uggaccuca                                                    19


<210>    45
<211>    19
<212>    RNA
<213>    Artificial Sequence

<220>
<223>    amphiregulin-specific siRNA


<400>    45
guuauuacag uccagcuua                                                    19


<210>    46
<211>    19
<212>    RNA
<213>    Artificial Sequence

<220>
<223>    amphiregulin-specific siRNA


<400>    46
uggaccucaa ugacaccua                                                    19


<210>    47
<211>    19
<212>    RNA
<213>    Artificial Sequence

<220>
<223>    amphiregulin-specific siRNA


<400>    47
cuggcuauau ugucgauga                                                    19


<210>    48
<211>    19
<212>    RNA

<213>    Artificial Sequence

<220>
<223>    amphiregulin-specific siRNA


<400>    48
gacggaaagu gaaaauacu                                                    19


<210>    49
<211>    19
<212>    RNA
<213>    Artificial Sequence

<220>
<223>    amphiregulin-specific siRNA


<400>    49
gcagccauag cugccuuua                                                    19


<210>    50
<211>    19
<212>    RNA
<213>    Artificial Sequence

<220>
<223>    amphiregulin-specific siRNA


<400>    50
ggagccgacu augacuacu                                                    19


<210>    51
<211>    19
<212>    RNA
<213>    Artificial Sequence

<220>
<223>    amphiregulin-specific siRNA


<400>    51
guaugauaac gaaccacaa                                                    19


<210>    52
<211>    19
<212>    RNA
<213>    Artificial Sequence

<220>
<223>    amphiregulin-specific siRNA


<400>    52
agugaaaugc cuucuagua                                                    19


<210>    53

<211>    19
<212>    RNA
<213>    Artificial Sequence

<220>
<223>    amphiregulin-specific siRNA


<400>    53
gauaacgaac cacaaauac                                                    19


<210>    54
<211>    19
<212>    RNA
<213>    Artificial Sequence

<220>
<223>    amphiregulin-specific siRNA


<400>    54
ugcauuagca gccauagcu                                                    19


<210>    55
<211>    19
<212>    RNA
<213>    Artificial Sequence

<220>
<223>    amphiregulin-specific siRNA


<400>    55
cgggagccga cuaugacua                                                    19


<210>    56
<211>    19
<212>    RNA
<213>    Artificial Sequence

<220>
<223>    amphiregulin-specific siRNA


<400>    56
ccgacuauga cuacucaga                                                    19


<210>    57
<211>    19
<212>    RNA
<213>    Artificial Sequence

<220>
<223>    amphiregulin-specific siRNA


<400>    57
gcauucacgg agaaugcaa                                                    19

<210>    58
<211>    19
<212>    RNA
<213>    Artificial Sequence

<220>
<223>    amphiregulin-specific siRNA


<400>    58
ccaugaaaac ucacagcau                                                    19


<210>    59
<211>    19
<212>    RNA
<213>    Artificial Sequence

<220>
<223>    amphiregulin-specific siRNA


<400>    59
cagcaugauu gacaguagu                                                    19


<210>    60
<211>    19
<212>    RNA
<213>    Artificial Sequence

<220>
<223>    amphiregulin-specific siRNA


<400>    60
cuuagaagac aauacguca                                                    19


<210>    61
<211>    19
<212>    RNA
<213>    Artificial Sequence

<220>
<223>    amphiregulin-specific siRNA


<400>    61
ugacuacuca gaagaguau                                                    19


<210>    62
<211>    19
<212>    RNA
<213>    Artificial Sequence

<220>
<223>    amphiregulin-specific siRNA


<400>    62

agaguugaac agguaguua                                                    19

<210>   63
<211>   19
<212>   RNA
<213>   Artificial Sequence

<220>
<223>   amphiregulin-specific siRNA

<400>   63
auauauagag caccuggaa                                                    19

<210>   64
<211>   19
<212>   RNA
<213>   Artificial Sequence

<220>
<223>   amphiregulin-specific siRNA

<400>   64
agcagccaua gcugccuuu                                                    19

<210>   65
<211>   19
<212>   RNA
<213>   Artificial Sequence

<220>
<223>   amphiregulin-specific siRNA

<400>   65
gaguugaaca gguaguuaa                                                    19

<210>   66
<211>   19
<212>   RNA
<213>   Artificial Sequence

<220>
<223>   amphiregulin-specific siRNA

<400>   66
cauucacgga gaaugcaaa                                                    19

<210>   67
<211>   19
<212>   RNA
<213>   Artificial Sequence

<220>
<223>   amphiregulin-specific siRNA

<400>    67
ucaggaaaua ugaaggaga                                                    19


<210>    68
<211>    19
<212>    RNA
<213>    Artificial Sequence

<220>
<223>    amphiregulin-specific siRNA


<400>    68
cagaagagua ugauaacga                                                    19


<210>    69
<211>    19
<212>    RNA
<213>    Artificial Sequence

<220>
<223>    amphiregulin-specific siRNA


<400>    69
gcuguuauua caguccagc                                                    19


<210>    70
<211>    19
<212>    RNA
<213>    Artificial Sequence

<220>
<223>    amphiregulin-specific siRNA


<400>    70
gggaagcgug aaccauuuu                                                    19


<210>    71
<211>    19
<212>    RNA
<213>    Artificial Sequence

<220>
<223>    amphiregulin-specific siRNA


<400>    71
agcugccuuu augucugcu                                                    19


<210>    72
<211>    19
<212>    RNA
<213>    Artificial Sequence

<220>

<223>    amphiregulin-specific siRNA

<400>    72
cacagugcug auggauuug                                                    19

<210>    73
<211>    19
<212>    RNA
<213>    Artificial Sequence

<220>
<223>    amphiregulin-specific siRNA

<400>    73
agucagaguu gaacaggua                                                    19

<210>    74
<211>    19
<212>    RNA
<213>    Artificial Sequence

<220>
<223>    amphiregulin-specific siRNA

<400>    74
uggaagcagu aacaugcaa                                                    19

<210>    75
<211>    19
<212>    RNA
<213>    Artificial Sequence

<220>
<223>    amphiregulin-specific siRNA

<400>    75
agaagaguau gauaacgaa                                                    19

<210>    76
<211>    19
<212>    RNA
<213>    Artificial Sequence

<220>
<223>    amphiregulin-specific siRNA

<400>    76
gaagcgugaa ccauuuucu                                                    19

<210>    77
<211>    19
<212>    RNA
<213>    Artificial Sequence

<220>
<223>    amphiregulin-specific siRNA


<400>    77
ggcuauauug ucgaugauu                                                    19


<210>    78
<211>    19
<212>    RNA
<213>    Artificial Sequence

<220>
<223>    amphiregulin-specific siRNA


<400>    78
gacaagagaa uggaaaugu                                                    19


<210>    79
<211>    19
<212>    RNA
<213>    Artificial Sequence

<220>
<223>    amphiregulin-specific siRNA


<400>    79
ugaaaugccu ucuaguagu                                                    19


<210>    80
<211>    19
<212>    RNA
<213>    Artificial Sequence

<220>
<223>    amphiregulin-specific siRNA


<400>    80
gugagugaaa ugccuucua                                                    19


<210>    81
<211>    19
<212>    RNA
<213>    Artificial Sequence

<220>
<223>    amphiregulin-specific siRNA


<400>    81
gaccucaaug acaccuacu                                                    19


<210>    82
<211>    19

<212>    RNA
<213>    Artificial Sequence

<220>
<223>    amphiregulin-specific siRNA


<400>    82
ccucaaugac accuacucu                                                    19


<210>    83
<211>    19
<212>    RNA
<213>    Artificial Sequence

<220>
<223>    amphiregulin-specific siRNA


<400>    83
gcugauggau uugagguua                                                    19


<210>    84
<211>    19
<212>    RNA
<213>    Artificial Sequence

<220>
<223>    amphiregulin-specific siRNA


<400>    84
ggaagcagua acaugcaaa                                                    19


<210>    85
<211>    19
<212>    RNA
<213>    Artificial Sequence

<220>
<223>    amphiregulin-specific siRNA


<400>    85
caguaacaug caaauguca                                                    19


<210>    86
<211>    19
<212>    RNA
<213>    Artificial Sequence

<220>
<223>    amphiregulin-specific siRNA


<400>    86
ggaccucaau gacaccuac                                                    19

<210>	87
<211>	19
<212>	RNA
<213>	Artificial Sequence

<220>
<223>	amphiregulin-specific siRNA


<400>	87
cagccauagc ugccuuuau	19


<210>	88
<211>	19
<212>	RNA
<213>	Artificial Sequence

<220>
<223>	amphiregulin-specific siRNA


<400>	88
uccaugaaaa cucacagca	19


<210>	89
<211>	19
<212>	RNA
<213>	Artificial Sequence

<220>
<223>	amphiregulin-specific siRNA


<400>	89
gcugccuuua ugucugcug	19


<210>	90
<211>	19
<212>	RNA
<213>	Artificial Sequence

<220>
<223>	amphiregulin-specific siRNA


<400>	90
gcucuugaua cucggcuca	19


<210>	91
<211>	19
<212>	RNA
<213>	Artificial Sequence

<220>
<223>	amphiregulin-specific siRNA


<400>	91
ugcugcugga uuggaccuc	19

<210>    92
<211>    19
<212>    RNA
<213>    Artificial Sequence

<220>
<223>    amphiregulin-specific siRNA


<400>    92
gaaccacaaa uaccuggcu                                                    19


<210>    93
<211>    19
<212>    RNA
<213>    Artificial Sequence

<220>
<223>    amphiregulin-specific siRNA


<400>    93
agagcaccug gaagcagua                                                    19


<210>    94
<211>    19
<212>    RNA
<213>    Artificial Sequence

<220>
<223>    amphiregulin-specific siRNA


<400>    94
caccuggaag caguaacau                                                    19


<210>    95
<211>    19
<212>    RNA
<213>    Artificial Sequence

<220>
<223>    amphiregulin-specific siRNA


<400>    95
cugaggaacg aaagaaacu                                                    19


<210>    96
<211>    19
<212>    RNA
<213>    Artificial Sequence

<220>
<223>    amphiregulin-specific siRNA

<400>    96
gugaaaugcc uucuaguag                                                    19


<210>    97
<211>    19
<212>    RNA
<213>    Artificial Sequence

<220>
<223>    amphiregulin-specific siRNA


<400>    97
cuacucuggg aagcgugaa                                                    19


<210>    98
<211>    19
<212>    RNA
<213>    Artificial Sequence

<220>
<223>    amphiregulin-specific siRNA


<400>    98
cugggaagcg ugaaccauu                                                    19


<210>    99
<211>    19
<212>    RNA
<213>    Artificial Sequence

<220>
<223>    amphiregulin-specific siRNA


<400>    99
acuacucaga agaguauga                                                    19


<210>    100
<211>    19
<212>    RNA
<213>    Artificial Sequence

<220>
<223>    amphiregulin-specific siRNA


<400>    100
caugcaaaug ucagcaaga                                                    19


<210>    101
<211>    19
<212>    RNA
<213>    Artificial Sequence

<220>
<223>    amphiregulin-specific siRNA

<400>    101
ugagguuacc ucaagaagu                                                    19


<210>    102
<211>    19
<212>    RNA
<213>    Artificial Sequence

<220>
<223>    amphiregulin-specific siRNA


<400>    102
acucggcuca ggccauuau                                                    19


<210>    103
<211>    19
<212>    RNA
<213>    Artificial Sequence

<220>
<223>    amphiregulin-specific siRNA


<400>    103
uucacggaga augcaaaua                                                    19


<210>    104
<211>    19
<212>    RNA
<213>    Artificial Sequence

<220>
<223>    amphiregulin-specific siRNA


<400>    104
cugcuggauu ggaccucaa                                                    19


<210>    105
<211>    19
<212>    RNA
<213>    Artificial Sequence

<220>
<223>    amphiregulin-specific siRNA


<400>    105
ugauucaguc agaguugaa                                                    19


<210>    106
<211>    19
<212>    RNA
<213>    Artificial Sequence

<220>
<223>    amphiregulin-specific siRNA


<400>    106
ucgacaagag aauggaaau                                                    19


<210>    107
<211>    19
<212>    RNA
<213>    Artificial Sequence

<220>
<223>    amphiregulin-specific siRNA


<400>    107
cucaagaagu gagaugucu                                                    19


<210>    108
<211>    19
<212>    RNA
<213>    Artificial Sequence

<220>
<223>    amphiregulin-specific siRNA


<400>    108
uucugcauuc acggagaau                                                    19


<210>    109
<211>    19
<212>    RNA
<213>    Artificial Sequence

<220>
<223>    amphiregulin-specific siRNA


<400>    109
agguuaccuc aagaaguga                                                    19


<210>    110
<211>    19
<212>    RNA
<213>    Artificial Sequence

<220>
<223>    amphiregulin-specific siRNA


<400>    110
aaugccuucu aguagugaa                                                    19


<210>    111
<211>    19
<212>    RNA

<213>    Artificial Sequence

<220>
<223>    amphiregulin-specific siRNA


<400>    111
augauucagu cagaguuga                                                    19


<210>    112
<211>    19
<212>    RNA
<213>    Artificial Sequence

<220>
<223>    amphiregulin-specific siRNA


<400>    112
aaacaagacg gaaagugaa                                                    19


<210>    113
<211>    19
<212>    RNA
<213>    Artificial Sequence

<220>
<223>    amphiregulin-specific siRNA


<400>    113
uuucugcauu cacggagaa                                                    19


<210>    114
<211>    19
<212>    RNA
<213>    Artificial Sequence

<220>
<223>    amphiregulin-specific siRNA


<400>    114
caaauaccug gcuauauug                                                    19


<210>    115
<211>    19
<212>    RNA
<213>    Artificial Sequence

<220>
<223>    amphiregulin-specific siRNA


<400>    115
gaagcaguaa caugcaaau                                                    19


<210>    116

<211>    19
<212>    RNA
<213>    Artificial Sequence

<220>
<223>    amphiregulin-specific siRNA


<400>    116
gacuacucag aagaguaug                                                    19


<210>    117
<211>    19
<212>    RNA
<213>    Artificial Sequence

<220>
<223>    amphiregulin-specific siRNA


<400>    117
gaugauucag ucagaguug                                                    19


<210>    118
<211>    19
<212>    RNA
<213>    Artificial Sequence

<220>
<223>    amphiregulin-specific siRNA


<400>    118
ugcauucacg gagaaugca                                                    19


<210>    119
<211>    19
<212>    RNA
<213>    Artificial Sequence

<220>
<223>    amphiregulin-specific siRNA


<400>    119
gcugaggaac gaaagaaac                                                    19


<210>    120
<211>    19
<212>    RNA
<213>    Artificial Sequence

<220>
<223>    amphiregulin-specific siRNA


<400>    120
uggauuugag guuaccuca                                                    19

<210>    121
<211>    19
<212>    RNA
<213>    Artificial Sequence

<220>
<223>    amphiregulin-specific siRNA


<400>    121
agugagaugu cuucaggga                                                    19


<210>    122
<211>    19
<212>    RNA
<213>    Artificial Sequence

<220>
<223>    amphiregulin-specific siRNA


<400>    122
gagguuaccu caagaagug                                                    19


<210>    123
<211>    19
<212>    RNA
<213>    Artificial Sequence

<220>
<223>    amphiregulin-specific siRNA


<400>    123
cuucgacaag agaauggaa                                                    19


<210>    124
<211>    19
<212>    RNA
<213>    Artificial Sequence

<220>
<223>    amphiregulin-specific siRNA


<400>    124
cucuugauac ucggcucag                                                    19


<210>    125
<211>    19
<212>    RNA
<213>    Artificial Sequence

<220>
<223>    amphiregulin-specific siRNA


<400>    125

gucagaguug aacagguag                                                    19


<210>    126
<211>    19
<212>    RNA
<213>    Artificial Sequence

<220>
<223>    amphiregulin-specific siRNA


<400>    126
caugaaaacu cacagcaug                                                    19


<210>    127
<211>    19
<212>    RNA
<213>    Artificial Sequence

<220>
<223>    amphiregulin-specific siRNA


<400>    127
ggaaauguac augcuauag                                                    19


<210>    128
<211>    19
<212>    RNA
<213>    Artificial Sequence

<220>
<223>    amphiregulin-specific siRNA


<400>    128
cauguaaugc agaauuuca                                                    19


<210>    129
<211>    19
<212>    RNA
<213>    Artificial Sequence

<220>
<223>    amphiregulin-specific siRNA


<400>    129
gauugacagu aguuuauca                                                    19


<210>    130
<211>    19
<212>    RNA
<213>    Artificial Sequence

<220>
<223>    amphiregulin-specific siRNA

<400>    130
guccagcuua gaagacaau                                                    19


<210>    131
<211>    19
<212>    RNA
<213>    Artificial Sequence

<220>
<223>    amphiregulin-specific siRNA


<400>    131
ugcuggauug gaccucaau                                                    19


<210>    132
<211>    19
<212>    RNA
<213>    Artificial Sequence

<220>
<223>    amphiregulin-specific siRNA


<400>    132
gaagacaaua cgucaggaa                                                    19


<210>    133
<211>    19
<212>    RNA
<213>    Artificial Sequence

<220>
<223>    amphiregulin-specific siRNA


<400>    133
aagacaauac gucaggaaa                                                    19


<210>    134
<211>    19
<212>    RNA
<213>    Artificial Sequence

<220>
<223>    amphiregulin-specific siRNA


<400>    134
ugaggaacga aagaaacuu                                                    19


<210>    135
<211>    19
<212>    RNA
<213>    Artificial Sequence

<220>

<223>    amphiregulin-specific siRNA

<400>    135
uucgacaaga gaauggaaa                                                    19

<210>    136
<211>    19
<212>    RNA
<213>    Artificial Sequence

<220>
<223>    amphiregulin-specific siRNA

<400>    136
ugcuguuauu acaguccag                                                    19

<210>    137
<211>    19
<212>    RNA
<213>    Artificial Sequence

<220>
<223>    amphiregulin-specific siRNA

<400>    137
gauggauuug agguuaccu                                                    19

<210>    138
<211>    19
<212>    RNA
<213>    Artificial Sequence

<220>
<223>    amphiregulin-specific siRNA

<400>    138
cucaaugaca ccuacucug                                                    19

<210>    139
<211>    19
<212>    RNA
<213>    Artificial Sequence

<220>
<223>    amphiregulin-specific siRNA

<400>    139
gguuaccuca agaagugag                                                    19

<210>    140
<211>    19
<212>    RNA
<213>    Artificial Sequence

<220>
<223>    amphiregulin-specific siRNA


<400>    140
gccgacuaug acuacucag                                                    19


<210>    141
<211>    19
<212>    RNA
<213>    Artificial Sequence

<220>
<223>    amphiregulin-specific siRNA


<400>    141
caaauauaua gagcaccug                                                    19


<210>    142
<211>    19
<212>    RNA
<213>    Artificial Sequence

<220>
<223>    amphiregulin-specific siRNA


<400>    142
agccgacuau gacuacuca                                                    19


<210>    143
<211>    19
<212>    RNA
<213>    Artificial Sequence

<220>
<223>    amphiregulin-specific siRNA


<400>    143
accuggcuau auugucgau                                                    19


<210>    144
<211>    19
<212>    RNA
<213>    Artificial Sequence

<220>
<223>    amphiregulin-specific siRNA


<400>    144
ccuacucugg gaagcguga                                                    19


<210>    145
<211>    19

<212>    RNA
<213>    Artificial Sequence

<220>
<223>    amphiregulin-specific siRNA


<400>    145
gcauuagcag ccauagcug                                                    19


<210>    146
<211>    19
<212>    RNA
<213>    Artificial Sequence

<220>
<223>    amphiregulin-specific siRNA


<400>    146
augauaacga accacaaau                                                    19


<210>    147
<211>    19
<212>    RNA
<213>    Artificial Sequence

<220>
<223>    amphiregulin-specific siRNA


<400>    147
gcgugaacca uuuucuggg                                                    19


<210>    148
<211>    19
<212>    RNA
<213>    Artificial Sequence

<220>
<223>    amphiregulin-specific siRNA


<400>    148
ugggaagcgu gaaccauuu                                                    19


<210>    149
<211>    19
<212>    RNA
<213>    Artificial Sequence

<220>
<223>    amphiregulin-specific siRNA


<400>    149
agcaguaaca ugcaaaugu                                                    19

<210> 150
<211> 19
<212> RNA
<213> Artificial Sequence

<220>
<223> amphiregulin-specific siRNA


<400> 150
ugaaaacuca cagcaugau 19


<210> 151
<211> 19
<212> RNA
<213> Artificial Sequence

<220>
<223> amphiregulin-specific siRNA


<400> 151
agacaauacg ucaggaaau 19


<210> 152
<211> 19
<212> RNA
<213> Artificial Sequence

<220>
<223> amphiregulin-specific siRNA


<400> 152
auacucggcu caggccauu 19


<210> 153
<211> 19
<212> RNA
<213> Artificial Sequence

<220>
<223> amphiregulin-specific siRNA


<400> 153
auuaugcugc uggauugga 19


<210> 154
<211> 19
<212> RNA
<213> Artificial Sequence

<220>
<223> amphiregulin-specific siRNA


<400> 154
ugcugaugga uuugagguu 19

<210>    155
<211>    19
<212>    RNA
<213>    Artificial Sequence

<220>
<223>    amphiregulin-specific siRNA


<400>    155
ugugagugaa augccuucu                                                    19


<210>    156
<211>    19
<212>    RNA
<213>    Artificial Sequence

<220>
<223>    amphiregulin-specific siRNA


<400>    156
auucagucag aguugaaca                                                    19


<210>    157
<211>    19
<212>    RNA
<213>    Artificial Sequence

<220>
<223>    amphiregulin-specific siRNA


<400>    157
aagggaggca aaaauggaa                                                    19


<210>    158
<211>    19
<212>    RNA
<213>    Artificial Sequence

<220>
<223>    amphiregulin-specific siRNA


<400>    158
uguuauuaca guccagcuu                                                    19


<210>    159
<211>    19
<212>    RNA
<213>    Artificial Sequence

<220>
<223>    amphiregulin-specific siRNA

<400>    159
acgaaagaaa cuucgacaa                                                    19


<210>    160
<211>    19
<212>    RNA
<213>    Artificial Sequence

<220>
<223>    amphiregulin-specific siRNA


<400>    160
cugcauucac ggagaaugc                                                    19


<210>    161
<211>    19
<212>    RNA
<213>    Artificial Sequence

<220>
<223>    amphiregulin-specific siRNA


<400>    161
agagaaugga aauguacau                                                    19


<210>    162
<211>    19
<212>    RNA
<213>    Artificial Sequence

<220>
<223>    amphiregulin-specific siRNA


<400>    162
cgucaggaaa uaugaagga                                                    19


<210>    163
<211>    19
<212>    RNA
<213>    Artificial Sequence

<220>
<223>    amphiregulin-specific siRNA


<400>    163
augcugcugg auuggaccu                                                    19


<210>    164
<211>    19
<212>    RNA
<213>    Artificial Sequence

<220>
<223>    amphiregulin-specific siRNA

<400>    164
aguaugauaa cgaaccaca                                                    19


<210>    165
<211>    19
<212>    RNA
<213>    Artificial Sequence

<220>
<223>    amphiregulin-specific siRNA


<400>    165
aaccacaaau accuggcua                                                    19


<210>    166
<211>    19
<212>    RNA
<213>    Artificial Sequence

<220>
<223>    amphiregulin-specific siRNA


<400>    166
aauauauaga gcaccugga                                                    19


<210>    167
<211>    19
<212>    RNA
<213>    Artificial Sequence

<220>
<223>    amphiregulin-specific siRNA


<400>    167
aaauugcauu agcagccau                                                    19


<210>    168
<211>    19
<212>    RNA
<213>    Artificial Sequence

<220>
<223>    amphiregulin-specific siRNA


<400>    168
acgaaccaca aauaccugg                                                    19


<210>    169
<211>    19
<212>    RNA
<213>    Artificial Sequence

<220>
<223>    amphiregulin-specific siRNA


<400>    169
aagcaguaac augcaaaug                                                    19


<210>    170
<211>    19
<212>    RNA
<213>    Artificial Sequence

<220>
<223>    amphiregulin-specific siRNA


<400>    170
cgcucuugau acucggcuc                                                    19


<210>    171
<211>    19
<212>    RNA
<213>    Artificial Sequence

<220>
<223>    amphiregulin-specific siRNA


<400>    171
guaacaugca aaugucagc                                                    19


<210>    172
<211>    19
<212>    RNA
<213>    Artificial Sequence

<220>
<223>    amphiregulin-specific siRNA


<400>    172
aauguacaug cuauagcau                                                    19


<210>    173
<211>    19
<212>    RNA
<213>    Artificial Sequence

<220>
<223>    amphiregulin-specific siRNA


<400>    173
cacggagaau gcaaauaua                                                    19


<210>    174
<211>    19
<212>    RNA

<213>    Artificial Sequence

<220>
<223>    amphiregulin-specific siRNA


<400>    174
cuguuauuac aguccagcu                                                    19


<210>    175
<211>    19
<212>    RNA
<213>    Artificial Sequence

<220>
<223>    amphiregulin-specific siRNA


<400>    175
cgacaagaga auggaaaug                                                    19


<210>    176
<211>    19
<212>    RNA
<213>    Artificial Sequence

<220>
<223>    amphiregulin-specific siRNA


<400>    176
aaaacaagac ggaaaguga                                                    19


<210>    177
<211>    19
<212>    RNA
<213>    Artificial Sequence

<220>
<223>    amphiregulin-specific siRNA


<400>    177
cuauauuguc gaugauuca                                                    19


<210>    178
<211>    19
<212>    RNA
<213>    Artificial Sequence

<220>
<223>    amphiregulin-specific siRNA


<400>    178
aguccaugaa aacucacag                                                    19


<210>    179

<211>    19
<212>    RNA
<213>    Artificial Sequence

<220>
<223>    amphiregulin-specific siRNA


<400>    179
gcugucgcuc uugauacuc                                                    19


<210>    180
<211>    19
<212>    RNA
<213>    Artificial Sequence

<220>
<223>    amphiregulin-specific siRNA


<400>    180
gcuauauugu cgaugauuc                                                    19


<210>    181
<211>    19
<212>    RNA
<213>    Artificial Sequence

<220>
<223>    amphiregulin-specific siRNA


<400>    181
cgaaagaaac uucgacaag                                                    19


<210>    182
<211>    19
<212>    RNA
<213>    Artificial Sequence

<220>
<223>    amphiregulin-specific siRNA


<400>    182
agcgugaacc auuuucugg                                                    19


<210>    183
<211>    19
<212>    RNA
<213>    Artificial Sequence

<220>
<223>    amphiregulin-specific siRNA


<400>    183
augaaaacuc acagcauga                                                    19

<210>    184
<211>    19
<212>    RNA
<213>    Artificial Sequence

<220>
<223>    amphiregulin-specific siRNA


<400>    184
acuucgacaa gagaaugga                                                    19


<210>    185
<211>    19
<212>    RNA
<213>    Artificial Sequence

<220>
<223>    amphiregulin-specific siRNA


<400>    185
caguccagcu uagaagaca                                                    19


<210>    186
<211>    19
<212>    RNA
<213>    Artificial Sequence

<220>
<223>    amphiregulin-specific siRNA


<400>    186
ugcaaauaua uagagcacc                                                    19


<210>    187
<211>    19
<212>    RNA
<213>    Artificial Sequence

<220>
<223>    amphiregulin-specific siRNA


<400>    187
agaagacaau acgucagga                                                    19


<210>    188
<211>    19
<212>    RNA
<213>    Artificial Sequence

<220>
<223>    amphiregulin-specific siRNA


<400>    188

gggagccgac uaugacuac                                                    19

<210>  189
<211>  19
<212>  RNA
<213>  Artificial Sequence

<220>
<223>  amphiregulin-specific siRNA

<400>  189
auggaaaugu acaugcuau                                                    19

<210>  190
<211>  19
<212>  RNA
<213>  Artificial Sequence

<220>
<223>  amphiregulin-specific siRNA

<400>  190
caaaaauugc auuagcagc                                                    19

<210>  191
<211>  19
<212>  RNA
<213>  Artificial Sequence

<220>
<223>  amphiregulin-specific siRNA

<400>  191
gugcugucgc ucuugauac                                                    19

<210>  192
<211>  19
<212>  RNA
<213>  Artificial Sequence

<220>
<223>  amphiregulin-specific siRNA

<400>  192
ugcugucgcu cuugauacu                                                    19

<210>  193
<211>  19
<212>  RNA
<213>  Artificial Sequence

<220>
<223>  amphiregulin-specific siRNA

<400>    193
uacucggcuc aggccauua                                                    19


<210>    194
<211>    19
<212>    RNA
<213>    Artificial Sequence

<220>
<223>    amphiregulin-specific siRNA


<400>    194
uggcuauauu gucgaugau                                                    19


<210>    195
<211>    19
<212>    RNA
<213>    Artificial Sequence

<220>
<223>    amphiregulin-specific siRNA


<400>    195
auucacggag aaugcaaau                                                    19


<210>    196
<211>    19
<212>    RNA
<213>    Artificial Sequence

<220>
<223>    amphiregulin-specific siRNA


<400>    196
aaacucacag caugauuga                                                    19


<210>    197
<211>    19
<212>    RNA
<213>    Artificial Sequence

<220>
<223>    amphiregulin-specific siRNA


<400>    197
auuacagucc agcuuagaa                                                    19


<210>    198
<211>    19
<212>    RNA
<213>    Artificial Sequence

<220>

<223>    amphiregulin-specific siRNA


<400>    198
ugauggauuu gagguuacc                                                    19


<210>    199
<211>    19
<212>    RNA
<213>    Artificial Sequence

<220>
<223>    amphiregulin-specific siRNA


<400>    199
ucagaagagu augauaacg                                                    19


<210>    200
<211>    19
<212>    RNA
<213>    Artificial Sequence

<220>
<223>    amphiregulin-specific siRNA


<400>    200
agaguaugau aacgaacca                                                    19


<210>    201
<211>    19
<212>    RNA
<213>    Artificial Sequence

<220>
<223>    amphiregulin-specific siRNA


<400>    201
ugauaacgaa ccacaaaua                                                    19


<210>    202
<211>    19
<212>    RNA
<213>    Artificial Sequence

<220>
<223>    amphiregulin-specific siRNA


<400>    202
acggagaaug caaauauau                                                    19


<210>    203
<211>    19
<212>    RNA
<213>    Artificial Sequence

<220>
<223>    amphiregulin-specific siRNA


<400>    203
cuaguaguga accguccuc                                                    19


<210>    204
<211>    19
<212>    RNA
<213>    Artificial Sequence

<220>
<223>    amphiregulin-specific siRNA


<400>    204
gaagaguaug auaacgaac                                                    19


<210>    205
<211>    19
<212>    RNA
<213>    Artificial Sequence

<220>
<223>    amphiregulin-specific siRNA


<400>    205
aaaugccuuc uaguaguga                                                    19


<210>    206
<211>    19
<212>    RNA
<213>    Artificial Sequence

<220>
<223>    amphiregulin-specific siRNA


<400>    206
uaacgaacca caaauaccu                                                    19


<210>    207
<211>    19
<212>    RNA
<213>    Artificial Sequence

<220>
<223>    amphiregulin-specific siRNA


<400>    207
auugucgaug auucaguca                                                    19


<210>    208
<211>    19

<212>    RNA
<213>    Artificial Sequence

<220>
<223>    amphiregulin-specific siRNA


<400>    208
acaugcaaau gucagcaag                                                    19


<210>    209
<211>    19
<212>    RNA
<213>    Artificial Sequence

<220>
<223>    amphiregulin-specific siRNA


<400>    209
uguugcuguu auuacaguc                                                    19


<210>    210
<211>    19
<212>    RNA
<213>    Artificial Sequence

<220>
<223>    amphiregulin-specific siRNA


<400>    210
uagagcaccu ggaagcagu                                                    19


<210>    211
<211>    19
<212>    RNA
<213>    Artificial Sequence

<220>
<223>    amphiregulin-specific siRNA


<400>    211
aacgaaagaa acuucgaca                                                    19


<210>    212
<211>    19
<212>    RNA
<213>    Artificial Sequence

<220>
<223>    amphiregulin-specific siRNA


<400>    212
aaauaccugg cuauauugu                                                    19

<210>    213
<211>    19
<212>    RNA
<213>    Artificial Sequence

<220>
<223>    amphiregulin-specific siRNA


<400>    213
aauacgucag gaaauauga                                                    19


<210>    214
<211>    19
<212>    RNA
<213>    Artificial Sequence

<220>
<223>    amphiregulin-specific siRNA


<400>    214
uggaaaugua caugcuaua                                                    19


<210>    215
<211>    19
<212>    RNA
<213>    Artificial Sequence

<220>
<223>    amphiregulin-specific siRNA


<400>    215
acgucaggaa auaugaagg                                                    19


<210>    216
<211>    19
<212>    RNA
<213>    Artificial Sequence

<220>
<223>    amphiregulin-specific siRNA


<400>    216
uuggaccuca augacaccu                                                    19


<210>    217
<211>    19
<212>    RNA
<213>    Artificial Sequence

<220>
<223>    amphiregulin-specific siRNA


<400>    217
uaacaugcaa augucagca                                                    19

<210>    218
<211>    19
<212>    RNA
<213>    Artificial Sequence

<220>
<223>    amphiregulin-specific siRNA


<400>    218
augacuacuc agaagagua                                                    19


<210>    219
<211>    19
<212>    RNA
<213>    Artificial Sequence

<220>
<223>    amphiregulin-specific siRNA


<400>    219
aaauccaugu aaugcagaa                                                    19


<210>    220
<211>    19
<212>    RNA
<213>    Artificial Sequence

<220>
<223>    amphiregulin-specific siRNA


<400>    220
ggaagcguga accauuuuc                                                    19


<210>    221
<211>    19
<212>    RNA
<213>    Artificial Sequence

<220>
<223>    amphiregulin-specific siRNA


<400>    221
ucugggaagc gugaaccau                                                    19


<210>    222
<211>    19
<212>    RNA
<213>    Artificial Sequence

<220>
<223>    amphiregulin-specific siRNA

<400>    222
uauugucgau gauucaguc                                                    19


<210>    223
<211>    19
<212>    RNA
<213>    Artificial Sequence

<220>
<223>    amphiregulin-specific siRNA


<400>    223
aguaacaugc aaaugucag                                                    19


<210>    224
<211>    19
<212>    RNA
<213>    Artificial Sequence

<220>
<223>    amphiregulin-specific siRNA


<400>    224
uuagaagaca auacgucag                                                    19


<210>    225
<211>    19
<212>    RNA
<213>    Artificial Sequence

<220>
<223>    amphiregulin-specific siRNA


<400>    225
aaaauccaug uaaugcaga                                                    19


<210>    226
<211>    19
<212>    RNA
<213>    Artificial Sequence

<220>
<223>    amphiregulin-specific siRNA


<400>    226
gcuggauugg accucaaug                                                    19


<210>    227
<211>    19
<212>    RNA
<213>    Artificial Sequence

<220>
<223>    amphiregulin-specific siRNA

<400>    227
cugauggauu ugagguuac                                                    19


<210>    228
<211>    19
<212>    RNA
<213>    Artificial Sequence

<220>
<223>    amphiregulin-specific siRNA


<400>    228
ccucaagaag ugagauguc                                                    19


<210>    229
<211>    19
<212>    RNA
<213>    Artificial Sequence

<220>
<223>    amphiregulin-specific siRNA


<400>    229
gagccgacua ugacuacuc                                                    19


<210>    230
<211>    19
<212>    RNA
<213>    Artificial Sequence

<220>
<223>    amphiregulin-specific siRNA


<400>    230
gauucaguca gaguugaac                                                    19